# EUROPEAN PATENT SPECIFICATION

(11) **EP 1 896 470 B1**
(45) Date of publication and mention of the grant of the patent: **26.05.2010**
(21) Application number: 06759904.3
(22) Date of filing: 15.05.2006
(51) Int. Cl.: C07D 471/04, A61K 31/437, A61P 35/00

(54) **PYRROLOPYRIDINE DERIVATIVES AS PROTEIN KINASE INHIBITORS**
PYRROLOPYRIDINDERIVATE ALS PROTEINKINASEINHIBITOREN
COMPOSES ET COMPOSITIONS EN TANT QU'INHIBITEURS DE PROTEINE KINASE

(30) Priority: 16.05.2005 US 681853 P
(43) Date of publication of application: 12.03.2008
(73) Proprietor: IRM LLC, Hamilton, HM 11 (BM); THE SCRIPPS RESEARCH INSTITUTE, La Jolla, CA 92037 (US)
(72) Inventor: OKRAM, Barun, San Diego, California 92122 (US); REN, Pingda, San Diego, California 92130 (US); GRAY, Nathanael S., Boston, Massachusetts 02130 (US)
(74) Representative: Kiddle, Simon John
(86) International application number: PCT/US2006/018868
(87) International publication number: WO 2006/124863

(56) References cited:
- WO-A-02/04447
- WO-A-98/47899
- WO-A-2004/016610
- WO-A-2005/095400
- WO-A-2006/050076

## Description

### BACKGROUND OF THE INVENTION

### Field of the Invention

The invention provides a novel class of compounds, pharmaceutical compositions comprising such compounds and methods of using such compounds to treat or prevent diseases or disorders associated with abnormal or deregulated kinase activity, particularly diseases or disorders that involve abnormal activation of the CDKs, Aurora, Jak2, Rock, CAMKII, FLT3, Tie2, TrkB, FGFR3 and KDR kinases.

### Background

The protein kinases represent a large family of proteins, which play a central role in the regulation of a wide variety of cellular processes and maintaining control over cellular function. A partial, non-limiting, list of these kinases include: tyrosine kinases such as FLT3, Tie2, TrkB, KDR and the fibroblast growth factor receptor, FGFR3; and serine/threonine kinases such as CDKs Aurora, Jak2, Rock and CAMKII. Aberrant kinase activity has been observed in many disease states including benign and malignant proliferative disorders as well as diseases resulting from inappropriate activation of the immune and nervous systems.

The novel compounds of this invention inhibit the activity of one or more protein kinases and are, therefore, expected to be useful in the treatment of kinase-associated diseases.
WO 2004/016610 describes compounds of the following general formula, which are said to have activity as inhibitors of the kinase Itk: 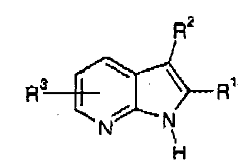 In this formula, the group R¹ represents a phenyl group.
WO 98/47899 describes compounds of the following general formula, which are said to have activity as inhibitors of the production of a number of inflammatory cytokines: 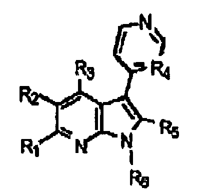 In this formula, the group R⁵ represents a phenyl group.
WO 02/04447 describes compounds of the following general formula, which are said to display anti-tumoral activity: 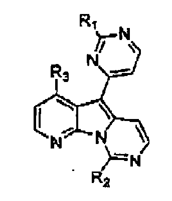 WO 2005/095400 was published on 13 October 2005 , after the priority date of the present application and before the filing date of the present application. It did enter the European Regional Phase, and therefore is of relevance under Article 54(3) EPC to those parts of the present application entitled to their priority date. This document describes compounds of the following general formula, which are said to have activity as inhibitors of JAK and other kinases:

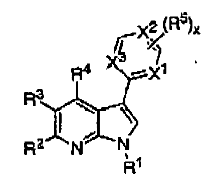

### SUMMARY OF THE INVENTION

In one aspect, the present invention provides compounds selected from Formula Ia, Ib and Ic: 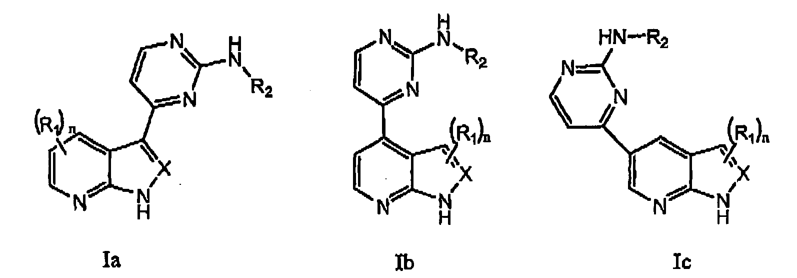 in which:
n is selected from 0,1 and 2;
R₁ is selected from halo, C₁₋₆alkyl, C₁₋₆alkoxy, halo-substituted-C₁₋₆alkyl and halo-substituted-C₁₋₆alkoxy;
R₂ is selected from C₆₋₁₀aryl-C₀₋₄alkyl and C₅₋₁₀heteroaryl-C₀₋₄alkyl; wherein said aryl or heteroaryl of R₂ is optionally substituted by 1-3 radicals independently selected from halo, C₁₋₆akl, C₁₋₆alkoxy, halo-substitubed-C₁₋₆alkyl, halo-substituted-C₁₋₆akoxy, -(O)₀₋₂R₅, -COOR₅, - C(O)NR₅R₆ and -NR₅C(O)R₆; wherein R₅ is selected from hydrogen and C₁₋₆alkyl; and R₆ is selected from C₆₋₁₀aryl and C₅₋₁₀heteroaryl; wherein said aryl or heteroaryl of R₆ is optionally substituted with 1 to 3 radicals independently selected from halo, C₁₋₆alkyl, C₁₋₆alkoxy, halo-substtuted-C₁₋₆alkyl and halo-substituted-C₁₋₆alkoxy;

X is selected from CR₇ or N; wherein R₇ is selected from hydrogen and C₁₋₆alkyl; and the pharmaceutically acceptable salts, hydrates and solvates thereof;
with the proviso that the compound is not 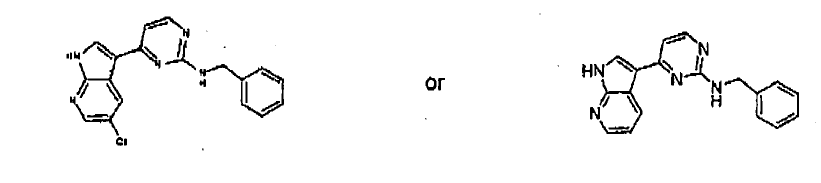 .

In a second aspect, the present invention provides a pharmaceutical composition which contains a therapeutically effective amount of a compound of Formula I or a pharmaceutically acceptable salt, hydrate or solvate thereof, in admixture with one or more suitable excipients.

Also described herein is a method of treating a disease in an animal in which inhibition of kinase activity, particularly CDKs, Aurora, Jak2, Rock, CAMKII, FLT3, Tie2, TrkB, FGFR3 and/or KDR. activity, can prevent, inhibit or ameliorate the pathology and/or symptomology of the diseases, which method comprises administering to the animal a therapeutically effective amount of a compound of Formula I or a N-oxide derivative, individual isomers and mixture of isomers thereof, or a pharmaceutically acceptable salt thereof .

In a third aspect, the present invention provides the use of a compound of Formula I in the manufacture of a medicament for treating a disease in an animal in which kinase activity, particularly CDKs, Aurora, Jack2, Rock, CAMKII, FLT3, Tie2, TrkB, FGFR3 and/or KDR activity, contributes to the pathology and/or symptomology of the disease.

In a fourth aspect, the present invention provides a process for preparing compounds of Formula I and the N-oxide derivatives, prodrug derivatives, protected derivatives, individual isomers and mixture of isomers thereof, and the pharmaceutically acceptable salts thereof.

### DETAILED DESCRIPTION OF THE INVENTION

### Definitions

"Alkyl" as a group and as a structural element of other groups, for example halo-substituted-alkyl and alkoxy, can be either straight-chained or branched. C₁₋₄-alkoxy includes, methoxy, ethoxy, and the like. Halo-substituted alkyl includes trifluoromethyl, pentafluoroethyl, and the like.

"Aryl" means a monocyclic or fused bicyclic aromatic ring assembly containing six to ten ring carbon atoms. For example, aryl may be phenyl or naphthyl, preferably phenyl. "Arylene" means a divalent radical derived from an aryl group.

"Heteroaryl" is as defined for aryl above where one or more of the carbon ring members indicated can be replaced by a heteroatom. For example C₅₋₈heteroaryl includes pyridyl, indolyl, indazolyl, quinoxalinyl, quinolinyl, benzofuranyl, benzopyranyl, benzothiopyranyl, benzo[1,3]dioxole, imidazolyl, benzo-imidazolyl, pyrimidinyl, furanyl, oxazolyl, isoxazolyl, triazolyl, tetrazolyl, pyrazolyl, thienyl, etc.

"Cycloalkyl" means a saturated or partially unsaturated, monocyclic, fused bicyclic or bridged polycyclic ring assembly containing the number of ring atoms indicated. For example, C₃₋₁₀cycloallcyl includes cyclopropyl, cyclobutyl, cyclopentyl, cyclohexyl, etc.

"Heterocycloalkyl" means cycloalkyl, as defined in this application, provided that one or more of the ring carbons indicated, are replaced by a moiety selected from -O-, -N=, -NR-, -C(O)-, -S-, -S(O) - or -S(O)₂-, wherein R is hydrogen, C₁₋₄alkyl or a nitrogen protecting group. For example, C₃₋₈heterocycloalkyl as used in this application to describe compounds of the invention includes morpholino, pyrrolidinyl, pyrrolidinyl-2-one, piperazinyl, piperidinyl, piperidinylone, 1,4-dioxa-8-aza-spiro[4.5]dec-8-yl, etc.

"Halogen" (or halo) preferably represents chloro or fluoro, but may also be bromo or iodo.

"Kinase Panel" is a list of kinases comprising Abl(human), Abl(T315I), JAK2, JAK3, ALK, JNKlal, ALK4, KDR, Aurora-A, Lck, Blk, MAPK1, Bmx, MAPKAP-K2, BRK, MEK1, CaMKII(rat), Met, CDK1/cyclinB, p70S6K, CHK2, PAK2, CK1, PDGFRα, CK2, PDK1, c-kit, Pim-2, c-RAF, PKA(h), CSK, PKBa, cSrc, PKCα, DYRK2, Plk3, EGFR, ROCK-I, Fes, Ron, FGFR3, Ros, Flt3, SAPK2α, Fms, SGK, Fyn, SIK, GSK3β, Syk, IGF-1R, Tie-2, IKKβ, TrKB, IR, WNK3, IRAK4, ZAP-70, ITK, AMPK(rat), LIMK1, Rsk2, Axl, LKB1, SAPK2β, BrSK2, Lyn (h), SAPK3, BTK, MAPKAP-K3, SAPK4, CaMKIV, MARK1, Snk, CDK2/cyclinA, MINK, SRPK1, CDK3/cyclinE, MKK4(m), TAK1, CDK5/p25, MKK6(h), TBK1, CDK6/cyclinD3, MLCK, TrkA, CDK7/cyclinH/MAT1, MRCKβ, TSSK1, CHK1, MSK1, Yes, CK1d, MST2, ZIPK, c-Kit (D816V), MuSK, DAPK2, NEK2, DDR2, NEK6, DMPK, PAK4, DRAK1, PAR-1Bα, EphA1, PDGFRβ, EphA2, Pim-1, EphA5, PKBβ, EphB2, PKCβI, EphB4, PKCδ, FGFR1, PKCη, FGFR2, PKCθ, FGFR4, PKD2, Fgr, PKG1β, Flt1, PRK2, Hck, PYK2, HIPK2, Ret, IKKα, RIPK2, IRR, ROCK-II(human), JNK2α2, Rse, JNK3, Rsk1(h), PI3 Kγ, PI3 Kδ and PI3-Kβ. Compounds of the invention are screened against the kinase panel (wild type and/or mutation thereof) and inhibit the activity of at least one of said panel members.

"Mutant forms of BCR-Abl" means single or multiple amino acid changes from the wild-type sequence. Mutations in BCR-ABL act by disrupting critical contact points between protein and inhibitor (for example, Gleevec, and the like), more often, by inducing a transition from the inactive to the active state, i.e. to a conformation to which BCR-ABL and Gleevec is unable to bind From analyses of clinical samples, the repertoire of mutations found in association with the resistant phenotype has been increasing slowly but inexorably over time. Mutations seem to cluster in four main regions. One group of mutations (G250E, Q252R, Y253F/H, E255K/V) includes amino acids that form the phosphate-binding loop for ATP (also known as the P-loop). A second group (V289A, F311L, T315I, F317L) can be found in the Gleevec binding site and interacts directly with the inhibitor via hydrogen bonds or Van der Waals' interactions. The third group of mutations (M351T, E355G) clusters in close proximity to the catalytic domain. The fourth group of mutations (H396R/P) is located in the activation loop, whose conformation is the molecular switch controlling kinase activation/inactivation. BCR-ABL point mutations associated with Gleevec resistance detected in CML and ALL patients include: M224Y, L248V, G250E, G250R, Q252R, Q252H, Y253H, Y253F, E255K, E255V, D276G, T277A, V289A, F311L, T315I, T315N, F317L, M343T, M315T, E355G, F359V, F359A, V379I, F382L, L387M, L387F, H396P, H396R, A397P, S4I7Y, E459K, and F486S (Amino acid positions, indicated by the single letter code, are those for the GenBank sequence, accession number AAB60394, and correspond to ABL type la; Martinelli et al., Haematologica/The Hematology Journal, 2005, April; 90-4). Unless otherwise stated for this invention, Bcr-Abl refers to wild-type and mutant forms of the enzyme.

"Treat", "treating" and "treatment" refer to a method of alleviating or abating a disease and/or its attendant symptoms.

### Description of the Preferred Embodiments

The present invention provides compounds and compositions for the treatment of kinase related disease, particularly CDKs, Aurora, Jak2, Rock, CAMKII, FLT3, Tie2, TrkB, FGFR3 and KDR kinase related diseases.

In one embodiment, with reference to compounds of Formula Ia, Ib and Ic, n is selected from 0 and 1; R₁ is C₁₋₆alkoxy; and R₂ is selected from C₆₋₁₀aryl-C₀₋₄akyl and C₅₋₁₀heteroaryl-C₀₋₄alkyl; wherein said aryl or heteroaryl of R₂ is optionally substituted by 1-3 radicals independently selected from halo, C₁₋₆alkyl, C₁₋₆alkoxy, halo-substituted-C₁₋₅alkyl, - S(O)₀₋₂R₅, -COOR₅, and -NR₅C(O)R₆; wherein R₅ is selected from hydrogen and C₁₋₆alkyl; and R₆ is C₆₋₁₀aryl optionally substituted with 1 to 3 radicals independently selected from halo-substituted-C₁₋₆alkyl.

In another embodiment, R₁ is methoxy; R₂ is selected from phenyl, benzyl and pyridinyl; wherein said phenyl, benzyl or pyridinyl of R₂ is optionally substituted with 1 to 2 radicals independently selected from chloro, bromo, fluoro, methyl, trifluoromethoxy, trifluoromethyl, -COO₂R₅, -S(O)₂R₅ and -NHC(O)R₆; wherein R₅ is selected from methyl and ethyl; and R₆ is phenyl optionally substituted with trifluoromethyl.

Preferred compounds of the invention are selected from (3-Chloro-phenyl)-[4-(1H-pyrrolo[2,3-b]pyridin-4-yl)-pyrimidin-2-yl]-amine; (4-Fluoro-phenyl)-[4-(1H-pyrrolo[2,3-b]pyridin-4-yl)-pyrimidin-2-yl]-amine; [4-(1H-Pyrrolo[2,3-b]pyridin-4-yl)-pyrimidin-2-yl]-(4-trifluoromethoxy-phenyl)-amine; [4-(1H-Pyrrolo[2,3-b]pyridin-4-yl)-pyrimidin-2-yl]-(3-trifluoromethyl-phenyl)-amine; (3,4-Difluoro-phenyl)-[4-(1H-pyrrolo[2,3-b]pyridin-4-yl)-pyrimidin-2-yl]-amine; [4-(1H-Pyrrolo[2,3-b]pyridin-4-yl)-pyrimidin-2-yl]-(4-trifluoromethyl-phenyl)-amine; 4-[4-(1H-Pyrrolo[2,3-b]pyridin-4-yl)-pyrimidin-2-ylamino]-benzoic acid ethyl ester; (3-Methoxy-phenyl)-[4-(1H-pyrrolo[2,3-b]pyridin-4-yl)-pyrimidin-2-yl]-amine; (3-Fluoro-benzyl)-[4-(1H-pyrrolo[2,3-b]pyridin-4-yl)-pyrimidin-2-yl]-amine; (3,5-Dimethoxy-phenyl)-[4-(1H-pyrrolo[2,3-b]pyridin-4-yl)-pyrimidin-2-yl]-amine; (2-Methyl-pyridin-4-yl)-[4-(1H-pyrrolo[2,3-b]pyridin-4-yl)-pyrimidin-2-yl]-amine; (2-Chloro-pyridin-4-yl)-[4-(1H-pyrrolo[2,3-b]pyridin-4-yl)-pyrimidin-2-yl]-amine; (2-Methoxy-pyridin-4-yl)-[4-(1H-pyrrolo[2,3-b]pyridin-4-yl)-pyrimidin-2-yl]-amine; (4-Methanesulfonyl-phenyl)-[4-(1H-pyrrolo[2,3-b]pyridin-4-yl)-pyrimidin-2-yl]-amine; Pyridin-4-yl-[4-(1H-pyrrolo[2,3-b]pyridin-4-yl)-pyrimidin-2-yl]-amine; (4-Methyl-pyrimidin-2-yl)-[4-(1H-pyrrolo[2,3-b]pyridin-4-yl)-pyrimidin-2-yl]-amine; (3-Bromo-phenyl)-[4-(1-methyl-1H-pyrrolo[2,3-b]pyridin-4-yl)-pyrimidin-2-yl]-amine; (3-Chloro-phenyl)-[4-(1-methyl-1H-pyrrolo[2,3-b]pyridin-4-yl)-pyrimidin-2-yl]-amine; [4-(1-Methyl-1H-pyrrolo[2,3-b]pyridin-4-yl)-pyrimidin-2-yl]-(3-trifluoromethylphenyl)-amine; (3-Bromo-4-methyl-phenyl)-[4-(1-methyl-1H-pyrrolo[2,3-b]pyridin-4-yl)-pyrimidin-2-yl]-amine; 2-{4-[2-(3-Bromo-phenylamino)-pyrimidin-4-yl]-pyrrolo[2,3-b]pyridin-1-yl}-ethanol; 2-{4-[2-(3-Trifluoromethyl-phenylamino)-pyrimidin-4-yl]-pyrrolo[2,3-b]pyridin-1-yl}-ethanol; 2-{4-[2-(3-Chloro-phenylamino)-pyrimidin-4-yl]-pyrrolo[2,3-b]pyridin-1-yl}-ethanol; 2-{4-[2-(3-Bromo-4-methyl-phenylamino)-pyrimidin-4-yl]-pyrrolo[2,3-b]pyridin-1-yl}-ethanol; {4-[1-(2-Amino-ethyl)-1H-pyrrolo[2,3-b]pyridin-4-yl]-pyrimidin-2-yl}-(3-bromo-phenyl)-amine; {4-[1-(2-Amino-ethyl)-1H-pyrrolo[2,3-b]pyridin-4-yl]-pyrimidin-2-yl}-(3-bromo-phenyl)-methyl-amine; {4-[1-(2-Amino-ethyl)-1H-pyrrolo[2,3-b]pyridin-4-yl]-pyrimidin-2-yl}-(4-trifluoromethyl-phenyl)-amine; N-{4-Methyl-3-[4-(1H-pyrrolo[2,3-b]pyridin-4-yl)-pyrimidin-2-ylamino]-phenyl}-3-trifluoromethyl-benzamide; [4-(1H-Pyrrolo[2,3-b]pyridin-3-yl)-pyrimidin-2-yl]-(4-trifluoromethyl-phenyl)-amine; (3,5-Dimethoxy-phenyl)-[4-(1H-pyrrolo[2,3-b]pyridin-3-yl)-pyrimidin-2-yl]-amine; (3,5-Difluoro-phenyl)-[4-(1H-pyrrolo[2,3-b]pyridin-3-yl)-pyrimidin-2-yl]-amine; (3-Bromo-phenyl)-[4-(1H-pyrrolo[2,3-b]pyridin-3-yl)-pyrimidin-2-yl]-amine; (3-Methoxy-phenyl)-[4-(1H-pyrrolo[2,3-b]pyridin-3-yl)-pyrimidin-2-yl]-amine; (4-Chlorophenyl)-[4-(1H-pyrrolo[2,3-b]pyridin-3-yl)-pyrimidin-2-yl]-amine; 4-[4-(1H-Pyrrolo[2,3-b]pyridin-3-yl)-pyrimidin-2-ylamino]-benzoic acid ethyl ester; N-{4-Methyl-3-[4-(1H-pyrrolo[2,3-b]pyrdin-3-yl)-pyrimidin-2-ylamino]-phenyl}-3-trifluoromethyl-benzamide; (3-Chloro-phenyl)-[4-(4-methoxy-1H-pyrrolo[2,3-b]pyridin-3-yl)-pyrimidin-2-yl]-amine; [4-(4-Methoxy-1H-pyrrolo[2,3-b]pyridin-3-yl)-pyrimidin-2-yl]-(3-trifluoromethyl-phenyl)-amine; (3-Bromo-phenyl)-[4-(4-methoxy-1H-pyrrolo[2,3-b]pyridin-3-yl)-pyrimidin-2-yl]-amine; N-{4-Methyl-3-[4-(1H-pyrrolo[2,3-b]pyridin-5-yl)-pyrimidin-2-ylamino]-phenyl}-3-trifluoromethyl-benzamide; N-Ethyl-4-[4-(1H-pyrrolo[2,3-b]pyridin-4-yl)-pyrimidin-2-ylamino]-benzenesulfonamide; N-(2-Methoxy-ethyl)-4-[4-(1H-pyrrolo[2,3-b]pyridin-4-yl)-pyrimidin-2-ylamino]-benzenesulfonamide; N-(3-Methoxy-propyl)-4-[4-(1H-pyrrolo[2,3-b]pyridin-4-yl)-pyrimidin-2-ylamino]-benzenesulfonamide; N-(3-Methoxy-propyl)-4-[4-(1H-pyrrolo[2,3-b]pyridin-3-yl)-pyrimidin-2-ylamino]-benzenesulfonamide; (3-Bromophenyl)-[4-(2-methyl-1H-pyrrolo[2,3-b]pyridin-4-yl)-pyrimidin-2-yl]-amine; (3-Chlorophenyl)-[4-(2-methyl-1H-pyrrolo[2,3-b]pyridin-4-yl)-pyrimidin-2-yl]-amine; [4-(2-Methyl-1H-pyrrolo[2,3-b]pyridin-4-yl)-pyrimidin-2-yl]-(3-trifluoromethyl-phenyl)-amine; N-(2-Methoxy-ethyl)-4-[4-(2-methyl-1H-pyrrolo[2,3-b]pyridin-4-yl)-pyrimidin-2-ylamino]-benzenesulfonamide; N-(3-Methoxy-propyl)-4-[4-(2-methyl-1H-pyrrolo[2,3-b]pyridin-4-yl)-pyrimidin-2-ylamino]-benzenesulfonamide; (3-Bromo-phenyl)-[4-(1H-pyrazolo[3,4-b]pyridin-4-yl)-pyrimidin-2-yl]-amine; (3-Chloro-phenyl)-[4-(1H-pyrazolo[3,4-b]pyridin-4-yl)-pyrimidin-2-yl]-amine; [4-(1H-Pyrazolo[3,4-b]pyridin-4-yl)-pyrimidin-2-yl]-(3-trifluoromethyl-phenyl)-amine; (3-Chloro-phenyl)-[4-(1H-pyrrolo[2,3-b]pyridin-5-yl)-pyrimidin-2-yl]-amine; (3-Bromo-phenyl)-[4-(1H-pyrrolo[2,3-b]pyridin-5-yl)-pyrimidin-2-yl]-amine; and [4-(1H-Pyrrolo[2,3-b]pyridin-5-yl)-pyrimidin-2-yl]-(3-trifluoromethylphenyl)-amine.

Further preferred compounds of the invention are detailed in the Examples and Table I, *infra.*

### Pharmacology and Utility

Compounds of the invention modulate the activity of kinases and, as such, are useful for treating diseases or disorders in which kinases, contribute to the pathology and/or symptomology of the disease. Examples of kinases that are inhibited by the compounds and compositions described herein and against which the methods described herein are useful include, but are not limited to, CDKs, Aurora, Jak2, Rock, CAMKII, FLT3, Tie2, TrkB, FGFR3 and KDR.

Abelson tyrosine kinase (i.e. Abl, c-Abl) is involved in the regulation of the cell cycle, in the cellular response to genotoxic stress, and in the transmission of information about the cellular environment through integrin signaling. Overall, it appears that the Abl protein serves a complex role as a cellular module that integrates signals from various extracellular and intracellular sources and that influences decisions in regard to cell cycle and apoptosis. Abelson tyrosine kinase includes sub-types derivatives such as the chimeric fusion (oncoprotein) BCR-Ab1 with deregulated tyrosine kinase activity or the v-Abl. BCR-Abl is critical in the pathogenesis of 95% of chronic myelogenous leukemia (CML) and 10% of acute lymphocytic leukemia. STI-571 (Gleevec) is an inhibitor of the oncogenic BCR-Abl tyrosine kinase and is used for the treatment of chronic myeloid leukemia (CML). However, some patients in the blast crisis stage of CML are resistant to STI-571 due to mutations in the BCR-Abl kinase. Over 22 mutations have been reported to date with the most common being G250E, E255V, T315I, F317L and M351T.

Compounds of the present invention inhibit abl kinase, especially v-abl kinase. The compounds of the present invention also inhibit wild-type BCR-Abl kinase and mutations of BCR-Abl kinase and are thus suitable for the treatment of Bcr-abl-positive cancer and tumor diseases, such as leukemias (especially chronic myeloid leukemia and acute lymphoblastic leukemia, where especially apoptotic mechanisms of action are found), and also shows effects on the subgroup of leukemic stem cells as well as potential for the purification of these cells *in vitro* after removal of said cells (for example, bone marrow removal) and reimplantation of the cells once they have been cleared of cancer cells (for example, reimplantation of purified bone marrow cells).

The Ras-Raf MEK-ERK signaling pathway mediates cellular response to growth signals. Ras is mutated to an oncogenic form in ∼ 15% of human cancer. The Raf family belongs to the serine/threonine protein kinase and it includes three members, A-Raf, B-Raf and c-Raf (or Raf-1). The focus on Raf being a drug target has centered on the relationship of Raf as a downstream effector of Ras. However, recent data suggests that B-Raf may have a prominent role in the formation of certain tumors with no requirement for an activated Ras allele (Nature 417, 949 - 954 (01 Jul 2002). In particular, B-Raf mutations have been detected in a large percentage of malignant melanomas.

Existing medical treatments for melanoma are limited in their effectiveness, especially for late stage melanomas. The compounds of the present invention also inhibit cellular processes involving b-Raf kinase, providing a new therapeutic opportunity for treatment of human cancers, especially for melanoma.

The compounds of the present invention also inhibit cellular processes involving c-Raf kinase. c-Raf is activated by the ras oncogene, which is mutated in a wide number of human cancers. Therefore inhibition of the kinase activity of c-Raf may provide a way to prevent ras mediated tumor growth [Campbell, S. L., Oncogene, 17, 1395 (1998)].

PDGF (Platelet-derived Growth Factor) is a very commonly occurring growth factor, which plays an important role both in normal growth and also in pathological cell proliferation, such as is seen in carcinogenesis and in diseases of the smooth-muscle cells of blood vessels, for example in atherosclerosis and thrombosis. Compounds of the invention can inhibit PDGF receptor (PDGFR) activity and are, therefore, suitable for the treatment of tumor diseases, such as gliomas, sarcomas, prostate tumors, and tumors of the colon, breast, and ovary.

Compounds of the present invention, can be used not only as a tumor-inhibiting substance, for example in small cell lung cancer, but also as an agent to treat non-malignant proliferative disorders, such as atherosclerosis, thrombosis, psoriasis, scleroderma and fibrosis, as well as for the protection of stem cells, for example to combat the hemotoxic effect of chemotherapeutic agents, such as 5-fluoruracil, and in asthma. Compounds of the invention can especially be used for the treatment of diseases, which respond to an inhibition of the PDGF receptor kinase.

Compounds of the present invention show useful effects in the treatment of disorders arising as a result of transplantation, for example, allogenic transplantation, especially tissue rejection, such as especially obliterative bronchiolitis (OB), i.e. a chronic rejection of allogenic lung transplants. In contrast to patients without OB, those with OB often show an elevated PDGF concentration in bronchoalveolar lavage fluids.

Compounds of the present invention are also effective in diseases associated with vascular smooth-muscle cell migration and proliferation (where PDGF and PDGF-R often also play a role), such as restenosis and atherosclerosis. These effects and the consequences thereof for the proliferation or migration of vascular smooth-muscle cells *in vitro* and *in vivo* can be demonstrated by administration of the compounds of the present invention, and also by investigating its effect on the thickening of the vascular intima following mechanical injury *in vivo.*

The trk family of neurotrophin receptors (trkA, trkB, trkC) promotes the survival, growth and differentiation of the neuronal and non-neuronal tissues. The TrkB protein is expressed in neuroendocrine-type cells in the small intestine and colon, in the alpha cells of the pancreas, in the monocytes and macrophages of the lymph nodes and of the spleen, and in the granular layers of the epidermis (Shibayama and Koizumi, 1996). Expression of the TrkB protein has been associated with an unfavorable progression of Wilms tumors and of neuroblastomas. TkrB is, moreover, expressed in cancerous prostate cells but not in normal cells. The signaling pathway downstream of the trk receptors involves the cascade of MAPK activation through the Shc, activated Ras, ERK-1 and ERK-2 genes, and the PLC-gammal transduction pathway (Sugimoto et al., 2001).

The kinase, c-Src transmits oncogenic signals of many receptors. For example, over-expression of EGFR or HER2/neu in tumors leads to the constitutive activation of c-src, which is characteristic for the malignant cell but absent from the normal cell. On the other hand, mice deficient in the expression of c-src exhibit an osteopetrotic phenotype, indicating a key participation of c-src in osteoclast function and a possible involvement in related disorders.

The Tec family kinase, Bmx, a non-receptor protein-tyrosine kinase, controls the proliferation of mammary epithelial cancer cells.

Fibroblast growth factor receptor 3 was shown to exert a negative regulatory effect on bone growth and an inhibition of chondrocyte proliferation. Thanatophoric dysplasia is caused by different mutations in fibroblast growth factor receptor 3, and one mutation, TDII FGFR3, has a constitutive tyrosine kinase activity which activates the transcription factor Stat1, leading to expression of a cell-cycle inhibitor, growth arrest and abnormal bone development (Su et al., Nature, 1997, 386, 288-292). FGFR3 is also often expressed in multiple myeloma-type cancers. Inhibitors of FGFR3 activity are useful in the treatment of T-cell mediated inflammatory or autoimmune diseases including but not limited to rheumatoid arthritis (RA), collagen II arthritis, multiple sclerosis (MS), systemic lupus erythematosus (SLE), psoriasis, juvenile onset diabetes, Sjogren's disease, thyroid disease, sarcoidosis, autoimmune uveitis, inflammatory bowel disease (Crohn's and ulcerative colitis), celiac disease and myasthenia gravis.

The activity of serum and glucocorticoid-regulated kinase (SGK), is correlated to perturbed ion-channel activities, in particular, those of sodium and/or potassium channels and compounds of the invention can be useful for treating hypertension.

Lin et al (1997) J. Clin. Invest. 100, 8: 2072-2078 and P. Lin (1998) PNAS 95, 8829-8834, have shown an inhibition of tumor growth and vascularization and also a decrease in lung metastases during adenoviral infections or during injections of the extracellular domain of Tie-2 (Tek) in breast tumor and melanoma xenograft models. Tie2 inhibitors can be used in situations where neovascularization takes place inappropriately (i.e. in diabetic retinopathy, chronic inflammation, psoriasis, Kaposi's sarcoma, chronic neovascularization due to macular degeneration, rheumatoid arthritis, infantile haemangioma and cancers).

Lck plays a role in T-cell signaling. Mice that lack the Lck gene have a poor ability to develop thymocytes. The function of Lck as a positive activator of T-cell signaling suggests that Lck inhibitors may be useful for treating autoimmune disease such as rheumatoid arthritis.

JNKs, along with other MAPKs, have been implicated in having a role in mediating cellular response to cancer, thrombin-induced platelet aggregation, immunodeficiency disorders, autoimmune diseases, cell death, allergies, osteoporosis and heart disease. The therapeutic targets related to activation of the JNK pathway include chronic myelogenous leukemia (CML), rheumatoid arthritis, asthma, osteoarthritis, ischemia, cancer and neurodegenerative diseases. As a result of the importance of JNK activation associated with liver disease or episodes of hepatic ischemia, compounds of the invention may also be useful to treat various hepatic disorders. A role for JNK in cardiovascular disease such as myocardial infarction or congestive heart failure has also been reported as it has been shown JNK mediates hypertrophic responses to various forms of cardiac stress. It has been demonstrated that the JNK cascade also plays a role in T-cell activation, including activation of the IL-2 promoter. Thus, inhibitors of JNK may have therapeutic value in altering pathologic immune responses. A role for JNK activation in various cancers has also been established, suggesting the potential use of JNK inhibitors in cancer. For example, constitutively activated JNK is associated with HTLV-1 mediated tumorigenesis [Oncogene 13:135-42 (1996)]. JNK may play a role in Kaposi's sarcoma (KS). Other proliferative effects of other cytokines implicated in KS proliferation, such as vascular endothelial growth factor (VEGF), IL-6 and TNFα, may also be mediated by JNK. In addition, regulation of the c-jun gene in p210 BCR-ABL transformed cells corresponds with activity of JNK, suggesting a role for JNK inhibitors in the treatment for chronic myelogenous leukemia (CML) [Blood 92:2450-60 (1998)].

Certain abnormal proliferative conditions are believed to be associated with raf expression and are, therefore, believed to be responsive to inhibition of raf expression. Abnormally high levels of expression of the raf protein are also implicated in transformation and abnormal cell proliferation. These abnormal proliferative conditions are also believed to be responsive to inhibition of raf expression. For example, expression of the c-raf protein is believed to play a role in abnormal cell proliferation since it has been reported that 60% of all lung carcinoma cell lines express unusually high levels of c-raf mRNA and protein. Further examples of abnormal proliferative conditions are hyper-proliferative disorders such as cancers, tumors, hyperplasia, pulmonary fibrosis, angiogenesis, psoriasis, atherosclerosis and smooth muscle cell proliferation in the blood vessels, such as stenosis or restenosis following angioplasty. The cellular signaling pathway of which raf is a part has also been implicated in inflammatory disorders characterized by T-cell proliferation (T-cell activation and growth), such as tissue graft rejection, endotoxin shock, and glomerular nephritis, for example.

The stress activated protein kinases (SAPKs) are a family of protein kinases that represent the penultimate step in signal transduction pathways that result in activation of the c-jun transcription factor and expression of genes regulated by c-jun. In particular, c-jun is involved in the transcription of genes that encode proteins involved in the repair of DNA that is damaged due to genotoxic insults. Therefore, agents that inhibit SAPK activity in a cell prevent DNA repair and sensitize the cell to agents that induce DNA damage or inhibit DNA synthesis and induce apoptosis of a cell or that inhibit cell proliferation.

Mitogen-activated protein kinases (MAPKs) are members of conserved signal transduction pathways that activate transcription factors, translation factors and other target molecules in response to a variety of extracellular signals. MAPKs are activated by phosphorylation at a dual phosphorylation motif having the sequence Thr-X-Tyr by mitogen-activated protein kinase kinases (MKKs). In higher eukaryotes, the physiological role of MAPK signaling has been correlated with cellular events such as proliferation, oncogenesis, development and differentiation. Accordingly, the ability to regulate signal transduction via these pathways (particularly via MKK4 and MKK6) could lead to the development of treatments and preventive therapies for human diseases associated with MAPK signaling, such as inflammatory diseases, autoimmune diseases and cancer.

The family of human ribosomal S6 protein kinases consists of at least 8 members (RSK1, RSK2, RSK3, RSK4, MSK1, MSK2, p70S6K and p70S6 Kb). Ribosomal protein S6 protein kinases play important pleotropic functions, among them is a key role in the regulation of mRNA translation during protein biosynthesis (Eur. J. Biochem 2000 November; 267(21): 6321-30, Exp Cell Res. Nov. 25, 1999; 253 (1):100-9, Mol Cell Endocrinol. May 25, 1999;151(1-2):65-77). The phosphorylation of the S6 ribosomal protein by p70S6 has also been implicated in the regulation of cell motility (Immunol. Cell Biol. 2000 August;78(4):447-51) and cell growth (Prog. Nucleic Acid Res. Mol. Biol., 2000;65:101-27), and hence, may be important in tumor metastasis, the immune response and tissue repair as well as other disease conditions.

The SAPK's (also called "jun N-terminal kinases" or "JNK's") are a family of protein kinases that represent the penultimate step in signal transduction pathways that result in activation of the c-jun transcription factor and expression of genes regulated by c-jun. In particular, c-jun is involved in the transcription of genes that encode proteins involved in the repair of DNA that is damaged due to genotoxic insults. Agents that inhibit SAPK activity in a cell prevent DNA repair and sensitize the cell to those cancer therapeutic modalities that act by inducing DNA damage.

BTK plays a role in autoimmune and/or inflammatory disease such as systemic lupus erythematosus (SLE), rheumatoid arthritis, multiple vasculitides, idiopathic thrombocytopenic purpura (ITP), myasthenia gravis, and asthma.. Because of BTK's role in B-cell activation, inhibitors of BTK are useful as inhibitors of B-cell mediated pathogenic activity, such as autoantibody production, and are useful for the treatment of B-cell lymphoma and leukemia.

CHK2 is a member of the checkpoint kinase family of serine/threonine protein kinases and is involved in a mechanism used for surveillance of DNA damage, such as damage caused by environmental mutagens and endogenous reactive oxygen species. As a result, it is implicated as a tumor suppressor and target for cancer therapy.

CSK influences the metastatic potential of cancer cells, particularly colon cancer.

Fes is a non-receptor protein tyrosine kinase that has been implicated in a variety of cytokine signal transduction pathways, as well as differentiation of myeloid cells. Fes is also a key component of the granulocyte differentiation machinery.

Flt3 receptor tyrosine kinase activity is implicated in leukemias and myelodysplastic syndrome. In approximately 25% of AML the leukemia cells express a constitutively active form of auto-phosphorylated (p) FLT3 tyrosine kinase on the cell surface. The activity of p-FLT3 confers growth and survival advantage on the leukemic cells. Patients with acute leukemia, whose leukemia cells express p-FLT3 kinase activity, have a poor overall clinical outcome. Inhibition of p-FLT3 kinase activity induces apoptosis (programmed cell death) of the leukemic cells.

Inhibitors of IKKα and IKKβ (1 & 2) are therapeutics for diseases which include rheumatoid arthritis, transplant rejection, inflammatory bowel disease, osteoarthritis, asthma, chronic obstructive pulmonary disease, atherosclerosis, psoriasis, multiple sclerosis, stroke, systemic lupus erythematosus, Alzheimer's disease, brain ischemia, traumatic brain injury, Parkinson's disease, amyotrophic lateral sclerosis, subarachnoid hemorrhage or other diseases or disorders associated with excessive production of inflammatory mediators in the brain and central nervous system.)

Met is associated with most types of the major human cancers and expression is often correlated with poor prognosis and metastasis. Inhibitors of Met are therapeutics for diseases which include cancers such as lung cancer, NSCLC (non small cell lung cancer), bone cancer, pancreatic cancer, skin cancer, cancer of the head and neck, cutaneous or intraocular melanoma, uterine cancer, ovarian cancer, rectal cancer, cancer of the anal region, stomach cancer, colon cancer, breast cancer, gynecologic tumors (e. g., uterine sarcomas, carcinoma of the fallopian tubes, carcinoma of the endometrium, carcinoma of the cervix, carcinoma of the vagina or carcinoma of the vulva), Hodgkin's Disease, cancer of the esophagus, cancer of the small intestine, cancer of the endocrine system (e. g., cancer of the thyroid, parathyroid or adrenal glands), sarcomas of soft tissues, cancer of the urethra, cancer of the penis, prostate cancer, chronic or acute leukemia, solid tumors of childhood, lymphocytic lymphomas, cancer of the bladder, cancer of the kidney or ureter (e. g., renal cell carcinoma, carcinoma of the renal pelvis), pediatric malignancy, neoplasms of the central nervous system (e. g., primary CNS lymphoma, spinal axis tumors, brain stem glioma or pituitary adenomas), cancers of the blood such as acute myeloid leukemia, chronic myeloid leukemia, etc, Barrett's esophagus (pre-malignant syndrome) neoplastic cutaneous disease, psoriasis, mycoses fungoides and benign prostatic hypertrophy, diabetes related diseases such as diabetic retinopathy, retinal ischemia and retinal neovascularization, hepatic cirrhosis, cardiovascular disease such as atherosclerosis, immunological disease such as autoimmune disease and renal disease. Preferably, the disease is cancer such as acute myeloid leukemia and colorectal cancer.

The Nima-related kinase 2 (Nek2) is a cell cycle-regulated protein kinase with maximal activity at the onset of mitosis that localizes to the centrosome. Functional studies have implicated Nek2 in regulation of centrosome separation and spindle formation. Nek2 protein is elevated 2- to 5-fold in cell lines derived from a range of human tumors including those of cervical, ovarian, prostate, and particularly breast.

p70S6K-mediated diseases or conditions include, but are not limited to, proliferative disorders, such as cancer and tuberous sclerosis.

There is growing evidence that kinase inhibitor therapy works consistently and reliably against cancers in which the kinase drug target is constitutively activated by gene mutations. There are multiple reports of mutations found in kinase resulting from a natural tumor selection process. A non-limiting list of examples of these would include: b-raf V599E mutant in over 60% of melanoma cases; Flt3-ITD mutants in 30% of AML cases; c-kit mutations in GIST patients; PDGFRα in GIST and HES; PDGFRβ in CMML, Pi3K mutants in colon and gastric cancers and glioblastomas; and EGFR mutants in 10% of lung cancers (Iressa® responsive) and in glioblastomas.

In accordance with the foregoing, described herein is a method for preventing or treating any of the diseases or disorders described above in a subject in need of such treatment, which method comprises administering to said subject a therapeutically effective amount (*See, "Administration and Pharmaceutical Compositions", infra*) of a compound of Formula I or a pharmaceutically acceptable salt thereof. For any of the above uses, the required dosage will vary depending on the mode of administration, the particular condition to be treated and the effect desired.

### Administration and Pharmaceutical Compositions

In general, compounds of the invention will be administered in therapeutically effective amounts via any of the usual and acceptable modes known in the art, either singly or in combination with one or more therapeutic agents. A therapeutically effective amount may vary widely depending on the severity of the disease, the age and relative health of the subject, the potency of the compound used and other factors. In general, satisfactory results are indicated to be obtained systemically at daily dosages of from about 0.03 to 2.5mg/kg per body weight. An indicated daily dosage in the larger mammal, e.g. humans, is in the range from about 0.5mg to about 100mg, conveniently administered, e.g. in divided doses up to four times a day or in retard form. Suitable unit dosage forms for oral administration comprise from ca. 1 to 50mg active ingredient.

Compounds of the invention can be administered as pharmaceutical compositions by any conventional route, in particular enterally, e.g., orally, e.g., in the form of tablets or capsules, or parenterally, e.g., in the form of injectable solutions or suspensions, topically, e.g., in the form of lotions, gels, ointments or creams, or in a nasal or suppository form. Pharmaceutical compositions comprising a compound of the present invention in free form or in a pharmaceutically acceptable salt form in association with at least one pharmaceutically acceptable carrier or diluent can be manufactured in a conventional manner by mixing, granulating or coating methods. For example, oral compositions can be tablets or gelatin capsules comprising the active ingredient together with a) diluents, e.g., lactose, dextrose, sucrose, mannitol, sorbitol, cellulose and/or glycine; b) lubricants, e.g., silica, talcum, stearic acid, its magnesium or calcium salt and/or polyethyleneglycol; for tablets also c) binders, e.g., magnesium aluminum silicate, starch paste, gelatin, tragacanth, methylcellulose, sodium carboxymethylcellulose and or polyvinylpyrrolidone; if desired d) disintegrants, e.g., starches, agar, alginic acid or its sodium salt, or effervescent mixtures; and/or e) absorbents, colorants, flavors and sweeteners. Injectable compositions can be aqueous isotonic solutions or suspensions, and suppositories can be prepared from fatty emulsions or suspensions. The compositions may be sterilized and/or contain adjuvants, such as preserving, stabilizing, wetting or emulsifying agents, solution promoters, salts for regulating the osmotic pressure and/or buffers. In addition, they may also contain other therapeutically valuable substances. Suitable formulations for transdermal applications include an effective amount of a compound of the present invention with a carrier. A carrier can include absorbable pharmacologically acceptable solvents to assist passage through the skin of the host. For example, transdermal devices are in the form of a bandage comprising a backing member, a reservoir containing the compound optionally with carriers, optionally a rate controlling barrier to deliver the compound to the skin of the host at a controlled and predetermined rate over a prolonged period of time, and means to secure the device to the skin. Matrix transdermal formulations may also be used. Suitable formulations for topical application, e.g., to the skin and eyes, are preferably aqueous solutions, ointments, creams or gels well-known in the art. Such may contain solubilizers, stabilizers, tonicity enhancing agents, buffers and preservatives.

Compounds of the invention can be administered in therapeutically effective amounts in combination with one or more therapeutic agents (pharmaceutical combinations). For example, synergistic effects can occur with other immunomodulatory or antiinflammatory substances, for example when used in combination with cyclosporin, rapamycin, or ascomycin, or immunosuppressant analogues thereof, for example cyclosporin A (CsA), cyclosporin G, FK-506, rapamycin, or comparable compounds, corticosteroids, cyclophosphamide, azathioprine, methotrexate, brequinar, leflunomide, mizoribine, mycophenolic acid, mycophenolate mofetil, 15-deoxyspergualin, immunosuppressant antibodies, especially monoclonal antibodies for leukocyte receptors, for example MHC, CD2, CD3, CD4, CD7, CD25, CD28, B7, CD45, CD58 or their ligands, or other immunomodulatory compounds, such as CTLA41g. Where the compounds of the invention are administered in conjunction with other therapies, dosages of the co-administered compounds will of course vary depending on the type of co-drug employed, on the specific drug employed, on the condition being treated and so forth.

The invention also provides for a pharmaceutical combinations, e.g. a kit, comprising a) a first agent which is a compound of the invention as disclosed herein, in free form or in pharmaceutically acceptable salt form, and b) at least one co-agent. The kit can comprise instructions for its administration.

The terms "co-administration" or "combined administration" or the like as utilized herein are meant to encompass administration of the selected therapeutic agents to a single patient, and are intended to include treatment regimens in which the agents are not necessarily administered by the same route of administration or at the same time.

The term "pharmaceutical combination" as used herein means a product that results from the mixing or combining of more than one active ingredient and includes both fixed and non-fixed combinations of the active ingredients. The term "fixed combination" means that the active ingredients, e.g. a compound of Formula I and a co-agent, are both administered to a patient simultaneously in the form of a single entity or dosage. The term "non-fixed combination" means that the active ingredients, e.g. a compound of Formula I and a co-agent, are both administered to a patient as separate entities either simultaneously, concurrently or sequentially with no specific time limits, wherein such administration provides therapeutically effective levels of the 2 compounds in the body of the patient. The latter also applies to cocktail therapy, e.g. the administration of 3 or more active ingredients.

### Processes for Making Compounds of the Invention

The present invention also includes processes for the preparation of compounds of the invention. In the reactions described, it can be necessary to protect reactive functional groups, for example hydroxy, amino, imino, thio or carboxy groups, where these are desired in the final product, to avoid their unwanted participation in the reactions. Conventional protecting groups can be used in accordance with standard practice, for example, see T.W. Greene and P. G. M. Wuts in "Protective Groups in Organic Chemistry", John Wiley and Sons, 1991.

Compounds of Formula I can be prepared by proceeding as in the following Reaction Scheme I:

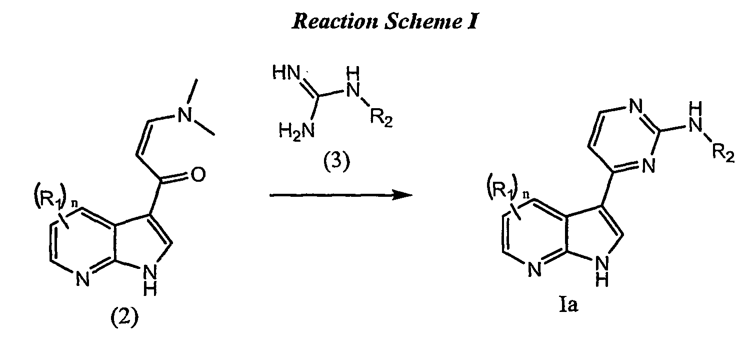

in which n, R₁ and R₂ are as defined in the Summary of the Invention. A compound of Formula Ia can be synthesized by reacting a compound of formula 2 with a compound of formula 3 in the presence of a suitable solvent (for example, sec-butanol, and the like). The reaction proceeds in a temperature range of about 20°C to about 80°C and can take up to about 24 hours to complete.

Compounds of Formula I can be prepared by proceeding as in the following Reaction Scheme II:

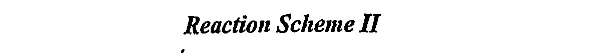

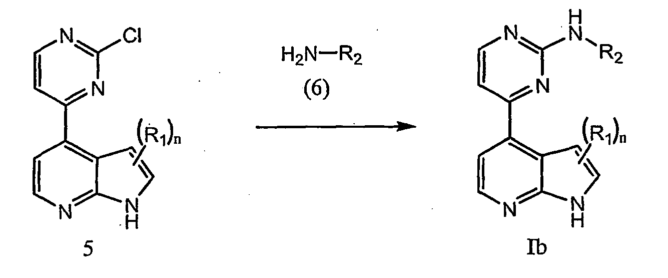

in which n, R₁ and R₂ are as defined in the Summary of the Invention. A compound of Formula Ib can be synthesized by reacting a compound of formula 5 with a compound of formula 6 in the presence of a suitable solvent (for example, sec-butanol, and the like) and a suitable catalyst (for example, p-toluenesulfonic acid monohydrate, and the like). The reaction proceeds in a temperature range of about 60°C to about 130°C and can take up to about 24 hours to complete.

Alternatively, compounds of Formula Ib can be synthesized by reacting a compound of formula 5 with a compound of formula 6 in the presence of a suitable solvent (for example, dioxane, and the like) and a suitable catalyst (for example, palladium acetate, and the like) and a suitable ligand (for example, XantPhos, and the like). The reaction proceeds in a temperature range of about 60°C to about 130°C and can take up to about 24 hours to complete.

Compounds of Formula I can be prepared by proceeding as in the following Reaction Scheme III: 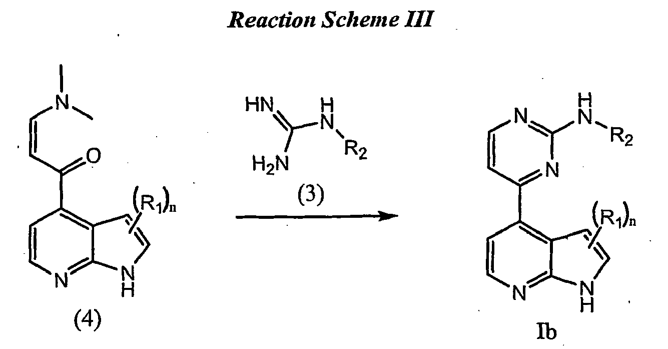 in which n, R₁ and R₂ are as defined in the Summary of the Invention. A compound of Formula I can be synthesized by reacting a compound of formula 4 with a compound of formula 3 in the presence of a suitable solvent (for example, sec-butanol, and the like). The reaction proceeds in a temperature range of about 20°C to about 80°C and can take up to about 24 hours to complete.

Detailed examples of the synthesis of a compound of Formula I can be found in the Examples, *infra.*

### Additional Processes for Making Compounds of the Invention

A compound of the invention can be prepared as a pharmaceutically acceptable acid addition salt by reacting the free base form of the compound with a pharmaceutically acceptable inorganic or organic acid. Alternatively, a pharmaceutically acceptable base addition salt of a compound of the invention can be prepared by reacting the free acid form of the compound with a pharmaceutically acceptable inorganic or organic base.

Alternatively, the salt forms of the compounds of the invention can be prepared using salts of the starting materials or intermediates.

The free acid or free base forms of the compounds of the invention can be prepared from the corresponding base addition salt or acid addition salt from, respectively. For example a compound of the invention in an acid addition salt form can be converted to the corresponding free base by treating with a suitable base (e.g., ammonium hydroxide solution, sodium hydroxide, and the like). A compound of the invention in a base addition salt form can be converted to the corresponding free acid by treating with a suitable acid (e.g., hydrochloric acid, etc.).

Compounds of the invention in unoxidized form can be prepared from N-oxides of compounds of the invention by treating with a reducing agent (e.g., sulfur, sulfur dioxide, triphenyl phosphine, lithium borohydride, sodium borohydride, phosphorus trichloride, tribromide, or the like) in a suitable inert organic solvent (e.g. acetonitrile, ethanol, aqueous dioxane, or the like) at 0 to 80°C.

Prodrug derivatives of the compounds of the invention can be prepared by methods known to those of ordinary skill in the art (e.g., for further details see Saulnier et al., (1994), Bioorganic and Medicinal Chemistry Letters, Vol. 4, p. 1985). For example, appropriate prodrugs can be prepared by reacting a non-derivatized compound of the invention with a suitable carbamylating agent (e.g., 1,1-acyloxyalkylcarbanochloridate, para-nitrophenyl carbonate, or the like).

Protected derivatives of the compounds of the invention can be made by means known to those of ordinary skill in the art. A detailed description of techniques applicable to the creation of protecting groups and their removal can be found in T. W. Greene, "Protecting Groups in Organic Chemistry", 3rd edition, John Wiley and Sons, Inc., 1999.

Compounds of the present invention can be conveniently prepared, or formed during the process of the invention, as solvates (e.g., hydrates). Hydrates of compounds of the present invention can be conveniently prepared by recrystallization from an aqueous/organic solvent mixture, using organic solvents such as dioxin, tetrahydrofuran or methanol.

Compounds of the invention can be prepared as their individual stereoisomers by reacting a racemic mixture of the compound with an optically active resolving agent to form a pair of diastereoisomeric compounds, separating the diastereomers and recovering the optically pure enantiomers. While resolution of enantiomers can be carried out using covalent diastereomeric derivatives of the compounds of the invention, dissociable complexes are preferred (e.g., crystalline diastereomeric salts). Diastereomers have distinct physical properties (e.g., melting points, boiling points, solubilities, reactivity, etc.) and can be readily separated by taking advantage of these dissimilarities. The diastereomers can be separated by chromatography, or preferably, by separation/resolution techniques based upon differences in solubility. The optically pure enantiomer is then recovered, along with the resolving agent, by any practical means that would not result in racemization. A more detailed description of the techniques applicable to the resolution of stereoisomers of compounds from their racemic mixture can be found in Jean Jacques, Andre Collet, Samuel H. Wilen, "Enantiomers, Racemates and Resolutions", John Wiley And Sons, Inc., 1981.

In summary, the compounds of Formula I can be made by a process, which involves:
(a) that of reaction schemes I, II or III; and
(b) optionally converting a compound of the invention into a pharmaceutically acceptable salt;
(c) optionally converting a salt form of a compound of the invention to a non-salt form;
(d) optionally converting an unoxidized form of a compound of the invention into a pharmaceutically acceptable N-oxide;
(e) optionally converting an N-oxide form of a compound of the invention to its unoxidized form;
(f) optionally resolving an individual isomer of a compound of the invention from a mixture of isomers;
(g) optionally converting a non-derivatized compound of the invention into a pharmaceutically acceptable prodrug derivative; and
(h) optionally converting a prodrug derivative of a compound of the invention to its non-derivatized form.

Insofar as the production of the starting materials is not particularly described, the compounds are known or can be prepared analogously to methods known in the art or as disclosed in the Examples hereinafter.

One of skill in the art will appreciate that the above transformations are only representative of methods for preparation of the compounds of the present invention, and that other well known methods can similarly be used.

### Examples

The present invention is further exemplified, but not limited, by the following examples that illustrate the preparation of compounds of Formula I according to the invention.

### Example 1

### 4-[2-(substituted-phenylamino)-pyrimidin-4-yl]-1H-pyrrolo[2,3-b]-pyridine

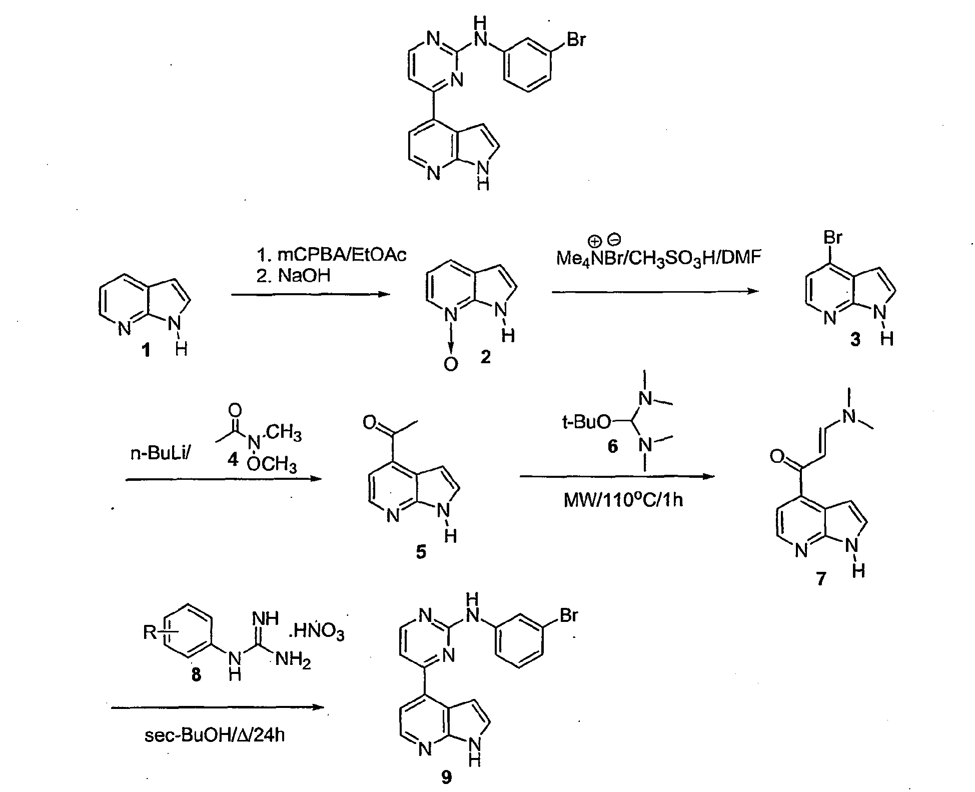

### Preparation of 1H-Pyrrolo[2,3-b]pyridine-7-oxide 2

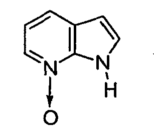

To a solution of 7-azaindole (10g, 84.6 mmol) in ethyl acetate (80 ml) is added mCPBA (18.96 g, 103.21 mmol) at 0°C for 30 minutes. The reaction mixture is brought to room temperature and stirred for 3 hours. It is then cooled at 0°C and stirred for 1 hour. The residue is filtered, washed with ethyl acetate and dried to afford N-oxide as a mCPBA salt.

A suspension of the above salt in water (80 ml) is cooled at 15°C and 30% K₂CO₃ is added to raise the pH to 10. After stirring for 1 hour at room temperature the reaction mixture is cooled at 0°C and kept stirring for 1 hour. The residue is filtered and washed with cold H₂O (10 ml). It is then dried to provide 7g of N-oxide.

### Preparation of 4-Bromo-1H-pyrrolo[2,3-b]pyridine (3)

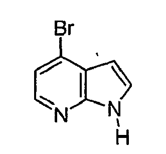

A suspension of 1H-pyrrolo[2,3-b]pyridine-7-oxide (6 g, 44.8 mmol) and tetramethyl-ammonium bromide (5.17, 34 mmol) in DMF (60 ml) is cooled at 0°C. After stirring for 15 minutes methanesulfonic anhydride (7.8 g, 44.8 mmol) is added at the same temperature. The suspension is brought to room temperature and stirred for 4 hours. The reaction mixture is then poured into water (100 ml) and the solution is neutralized with 50% NaOH. The solution is cooled to between 0 and 10°C. The resultant solid is then filtered and dried to afford 4-bromo-7-azaindole: ¹H NMR 600 MHz (DMSO-*d*₆) δ 10.81 (brs, 1H), 8.3 6 (d, *J* = 3.2 Hz, 1H), 7.63 (*d, J* = 3.2 Hz, 1H), 7.52 (*d, J =* 4.1 Hz, 1H), 6.79 (*d*, *J* = 3.6 Hz, 1H); MS *m*/*z* 198.1 (M+ 1).

### Preparation of 4-Acetyl-1H-pyrrolo[2,3-b]pyridine (5)

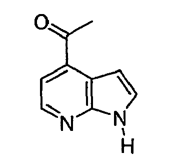

4-Bromo-7-azaindole (1 g, 5 mmol) is dissolved in THF (15 ml) and cooled at -78°C. n-BuLi (5.25 ml, 2 M solution, 2.1eq.) is added slowly at the same temperature over 10 minutes. After stirring for 45 minutes, N-methoxy-N-methyl-acetamide (1.1 ml, 10.5 mmol) is added slowly at the same temperature. The reaction mixture is brought back to room temperature and stirred for 3 hours. It is then quenched by adding saturated NH₄Cl (2 ml) at -78°C. The reaction mixture is worked up with ethyl acetate and the organic layer is washed with brine and dried over NaSO₄. The evaporation of the solvent under vacuo resulted in crude compound which is purified by silica gel column chromatography using hexane/ethyl acetate as an eluent: ¹H NMR 600 MHz (DMSO-d₆) δ 11.02 (brs, 1H), 8.41 (d, J = 3.2 Hz, 1H), 7.53 (d, J = 3.2 Hz, 1H), 7.54 (d, J = 4.4 Hz, 1H), 6.09 (d, J = 3.5 Hz, 1H), 2.47 (s, 3H); MS m/z 161.1 (M + 1).

### Preparation of Enaminone (7)

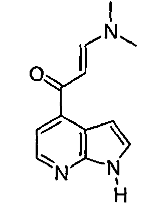

A mixture of 4-Acetyl-7-azaindole (1g, 6.2 mmol) and Bredereck reagent (2.56 ml, 12.4 mmol) is irradiated by microwave at 110°C for 1 hour. The excess reagent is removed under vacuo to afford enaminone which is used for the next step without further purification.

### Preparation of 4-[2-(substituted-phenylamino)-pyrimidin-4-yl]-1H-pyrrolo[2,3-b]-pyridine (9).

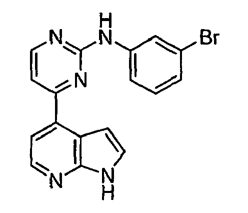

A mixture of enaminone (35 mg, 0.16 mmol), 3-bromophenylguanidine nitrate (48.57 mg, 0.178 mmol), LiOH (11.5 mg, 48 mmol) in sec-butanol (1 ml) is heated at 130°C for 24 hours. Solvent is removed by vacuo and the resulting crude solid is purified by subjecting to reverse-phase LC-MS to yield of the title compound: ¹H NMR 600 MHz (DMSO-d₆) δ 11.92 (brs, 1H), 9.83 (s, 1H), 8.65 (d, J = 4.8 Hz, 1H), 8.38 (d, J = 4.8 Hz, 1H), 8.12 (t, J = 2 Hz, 1H), 7.77 (m, 1H), 7.69 (m, 1H), 7.65 (d, J =4.8 Hz, 1H), 7.59 (t, J = 2 Hz, 1H), 7.50 (d, J = 5.2 Hz, 1H), 7.33 (t, J = 8.4 Hz, 1H), 7.06-7.07 (m, 1H); MS m/z 366.2 (M + 1).

### Example 2a and 2b

### Pyridin-2-yl-[4-(1H-pyrrolo[2,3-b]pyridin-4-yl)-pyrimidin-2-yl]-amine (2a) & (3-Bromo-4-methyl-phenyl)-[4-(1H-pyrrolo[2,3-b]pyridin-4-yl)-pyrimidin-2-yl]-amine (2b)

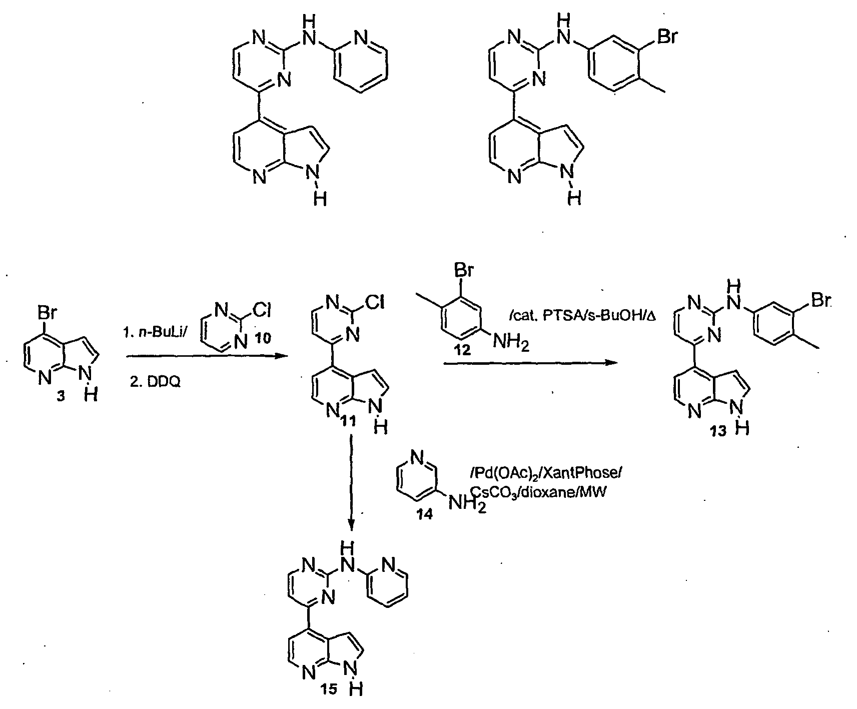

### Preparation of 4-[2-Chloro-pyrimidin-4-yl]-1H-pyrrolo[2,3-b]pyridine (11)

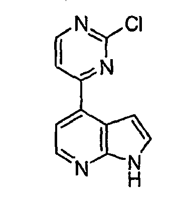

To a solution of 4-Bromo-7-azaindole (500mg, 2.5 mmol) THF is added n-BuLi (2.6 ml, 2 M solution in hexanes, 2.1 eqv) at -78°C. The reaction mixture is kept stirring at the same temperature for 45 minutes. 2-Chloropyrimidine (314.9 mg, 2.75 mmol) in THF is then added in dropwise at -78°C. After stirring for another 2 hours, 1ml of water is added and stirring is continued for another 20 minutes. DDQ (618.7mg, 2.75 mmol) in THF is then added at the same temperature and the reaction mixture is adjusted to 0°C and stirred for 1 hour. It is quenched with 1 N NaOH and worked up with ethyl acetate. The organic layer is washed with saturated NaHCO₃ solution, brine and water. The combined organic solvent is dried (MgSO₄) and evaporated to yield the crude 4-pyrimidine substituted azaindole compound. The compound is purified by silica gel column chromatography using hexane/ethyl acetate as eluent: ¹H NMR 600 MHz (DMSO-d₆) δ 12.05 (brs, 1H), 8.91 (d, J = 5.2 Hz, 1H), 8.41 (d, J = 4.8 Hz, 1H), 8.25 (d, J = 5.2 Hz, 1H), 7.76 (d, J = 5.2 Hz, 1H), 7.72 (brs, 1H), 7.09 (brs, 1H); MS m/z 231.0 (M + 1).

### Preparation of (3-Bromo-4-methyl-phenyl)-[4-(1H-pyrrolo[2,3-b]pyridin-4-yl)-pyrimidin-2-yl]-amine (13)

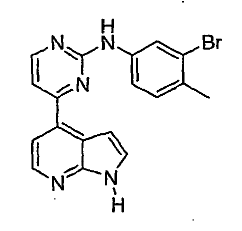

A Smith vial (2-5 mL) is charged with 11 (26.5 mg, 0.115 mmol), 3-bromo-4-methylaniline (42.54 mg, 0.23 mmol) and p-toluenesulfonic acid monohydrate (4.4mg, 0.023 mmol), sec-BuOH (0.5 mL). After purging with Argon, the vial is sealed and irradiated at 100°C for 1.5 hours in a Smith Synthesizer. The resulting solution is subjected to purification by reverse-phase LC-MS to yield the title compound: MS m/z 380.04 (M + 1).

### Preparation of Pyridin-2-yl-[4-(1H-pyrrolo[2,3-b]pyridin-4-yl)-pyrimidin-2-yl]-amine (15)

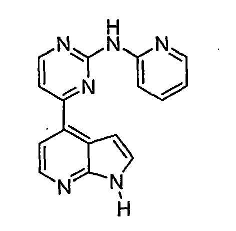

To a solution of 4-[2-Chloro-pyrimidin-4-yl]-1H-pyrrolo[2,3-b]pyridine (26.5 mg, 0.12 mmol) in dioxane (1 mL) is added Pd(OAc)₂ (2.6 mg, 0.0115 mM), XantPhose (9.98 mg, 0.017 mmol), CsCO₃ (112.40 mg, 0.345 mmol) and 2-aminopyridine (16.17 mg, 0.172 mmol). The vial is purged with Argon, capped and subjected to microwave radiation for 10 minutes at 150°C. It is then filtered and evaporated in vacuo. The desired compound is separated by reverse-phase LC-MS to yield the title compound: ¹H NMR 600 MHz (DMSO-d₆) δ 11.97 (brs, 1H), 11.55 (brs, 1H), 8.79 (d, J = 4.8 Hz, 1H), 8.37-8.35 (m, 2H), 8.12 (d, J = 4.8 Hz, 1H), 7.84-7.83 (m, 2H), 7.70 (d, J = 4.8 Hz, 1H), 7.65 (d, J =4.8 Hz, 1H), 7.25-7.20 (m, 2H); (M + 1); MS m/z 289.10 (M + 1).

### Example 3

### (3-Chloro-phenyl)-[4-(1H-pyrrolo[2,3-b]pyridin-3-yl)-pyrimidin-2-yl]-amine

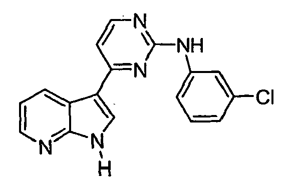

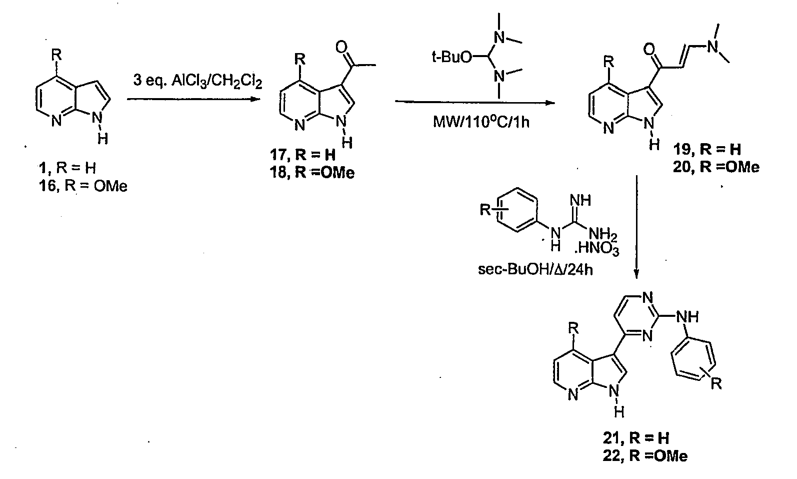

### 4-Methoxy-1H-pyrrolo[2,3-b]pyridine (16)

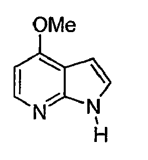

4-Methoxy-1H-pyrrolo[2,3-b]pyridine is synthesized from 7-azaindole using the reported procedure. [Cottan, H. B.; Girfis, N. S.; Robins, R. K. Journal of Heterocyclic Chemistry (1989), 26(2), 317-25].

### 3-Acetyl-1H-pyrrolo[2,3-b]pyridine (17)

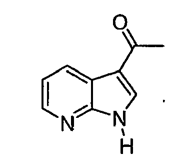

7-Azaindole (1 g, 8.4 mmol) is dissolved in dichloromethane and the solution is added onto a suspension of AlCl₃ (5.6g, 42 mmol) in dichloromethane at room temperature. After stirring for 1 hour, acetyl chloride (1.8 ml, 25.2 mmol) is added slowly at the same temperature and kept stirring for 2 hours. After completion (analytical HPLC), the reaction mixture is cooled to 0°C and 3 ml of methanol is added carefully in order to quench the reaction. The reaction mixture is concentrated and the residue is washed with hexanes. The residue is then neutralized with 30% NaOH solution and it is extracted with dichloromethane. The filtrate is washed (brine), dried (MgSO₄) and evaporated to yield crude product. The pure product is obtained by re-crystallizing with hexanes/dichloromethane.

### Preparation of Enaminone (19)

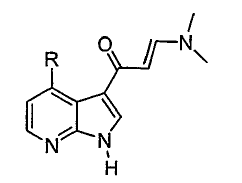

A mixture of 3-Acetyl-7-azaindole (1g, 6.2 mmol) and Bredereck reagent (2 .5equiv) is irradiated by microwave at 110°C for 1 hour. The excess reagent is removed under vacuo to afford enaminone which is used without further purification.

### Preparation of (3-Chlorophenyl)-[4-(1H-pyrrolo[2,3-b]pyridine-3-yl)-pyrimidin-2-yl]-amine (21)

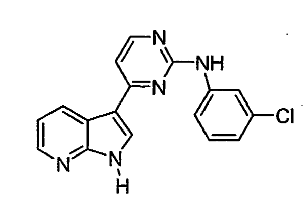

A mixture of enaminone (35 mg, 0.16 mmol), 3-chlorophenylguanidine nitrate (41.29 mg, 0.178 equiv) , LiOH (11.5 mg, 48 mmol) in sec-butanol (1 ml)is heated at 130°C for 24 hours. Solvent is removed and the residue is washed with H₂O and hexane to yield desired compound. The compound is purified by subjecting to reverse-phase LC-MS to yield the title compound: ¹H NMR 600 MHz (DMSO-d₆) δ 12.41 (s, 1H), 9.71 (s, 1H), 8.90 (d, J = 7.6 Hz, 1H), 8.53 (d, J = 2.8, 1H), 8.38 (d, J = 5.6 Hz, 1H), 8.33 (dd, J = 4.4,1.6 Hz, 1H), 7.85-7.83 (m, 2H), 7.39 (d, J = 2.4 Hz, 2H), 7.38 (s, 1H), 7.24 (dd, J = 7.6, 4.4 Hz, 1H); MS m/z 322.14 (M + 1).

By repeating the procedures described in the above examples, using appropriate starting materials, the following compounds of Formula I, as identified in Table 1, are obtained.

**Table 1**

| **Compound Number** | **Structure** | **Physical Data** ¹H NMR 400 MHz (DMSO-*d₆*) and/or MS (*m*/*z*) |
|---|---|---|
| 1 | 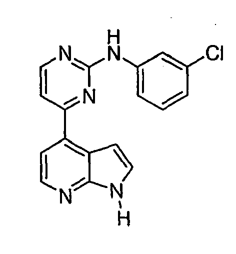 | δ 11.88 (brs, 1H), 9.91 (s, 1H), 8.62 (*d*, *J* = 4.8 Hz, 1H), 8.32 (*d*, J = 4.8 Hz, 1H), 8.06 (*t, J* = 2 Hz, 1H), 7.66 (m, 1H), 7.65 (m, 1H), 7.61 (d, J =4.8 Hz, 1H), 7.59 (t, *J* = 2 Hz, 1H), 7.49 (d, *J* = 5.2 Hz, 1H), 7.26 (t, *J* = 8.4 Hz, 1H), 7.06-7.07 (brs, 1H); MS *m*/*z* 322.1 (M + 1). |
| 2 | 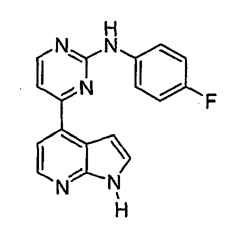 | δ 11.87 (brs, 1H), 9.71 (s, 1H), 8.55 (*d, J* = 5.2 Hz, 1H), 8.31 (*d*, J = 4.8 Hz, 1H), 7.78-7.75 (m, 2H), 7.61 (d, J = 5.2 Hz, 1H), 7.58 (t, J = 3.2 Hz, 1H), 7.42 (d, J = 5.2Hz, 1H), 7.12-6.98 (m, 3H); MS 306.11 *m*/*z* (M + 1). |
| 3 | 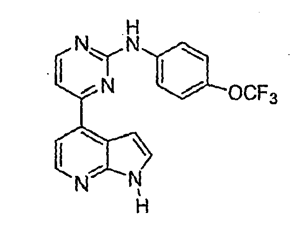 | δ 11.88 (brs, 1H), 9.90 (s, 1H), 8.60 (*d*, *J* = 4.8 Hz, 1H), 8.32 (*d*, *J* = 4.8 Hz, 1H), 7.89 (d, *J* = 2.4 Hz, 1H), 7.87 (d, *J* = 2.1 Hz, 1H), 7.62 (d, *J* = 5.2 Hz, 1H), 7.59 (t, *J* = 3.2 Hz, 1H), 7.26-7.24 (m, 2H), 7.04-7.03 (m, 2H),; MS *m*/*z* 372.09(M + 1). |
| 4 | 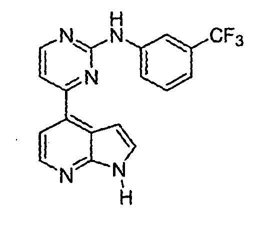 | δ 11.85 (brs, 1H), 10.01 (s, 1H), 8.59 (*d*, *J* = 4.8 Hz, 1H), 8.30-8.29 (*m*, 1H), 8.27 (d, *J* = 4.8 Hz, 1H), 7.94 (d, J = 7.6 Hz, 1H), 7..59 (d, *J* = 4.8, 1H), 7.53-7.52 (m, 1H), 7.47 (d, *J* = 4.8 Hz, 1H), 7.42 (t, *J* = 7.6 Hz, 1H), 7.18 (d, *J* = 7.6 Hz, 1H), 6.99 (m, 1H),; MS *m*/*z* 356.12 (M + 1). |
| 5 | 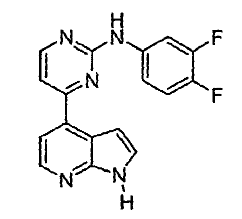 | MS *m*/*z* 324.2 (M + 1). |
| 6 | 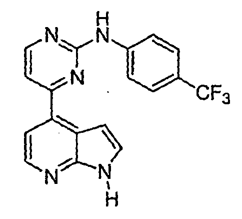 | MS *m*/*z* 356.15 (M + 1). |
| 7 | 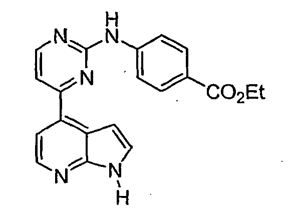 | MS *m*/*z* 360.13 (M+ 1). |
| 8 | 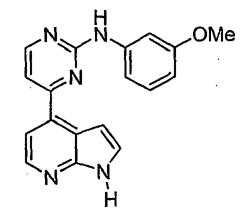 | MS *m*/*z* 318.12 (M + 1). |
| 9 | 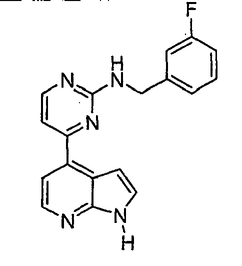 | MS *m*/*z* 320.2 (M+ 1). |
| 10 | 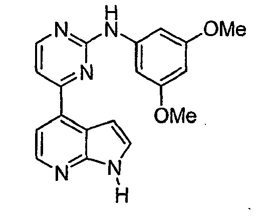 | MS *m*/*z* 348.1(M+ 1). |
| 11 | 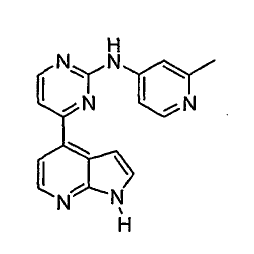 | δ 12.04 (brs, 1H), 11.39 (s, 1H), 8.91 (*d*, *J* = 5.2 Hz, 1H), 8.52 (*d*, *J* = 6.8 Hz, 1H), 8.44 (*d*, *J* = 4.8 Hz, 1H), 8.27-8.22 (*m*, 1H), 8.09-8.08 (m, 1H), 7.90 (d, J = 5.2 Hz, 1H), 7.75-7.72 (m, 2H), 7.14 (m, 1H), 2.62 (s, 3H); .MS *m*/*z* 303.13 (M + 1). |
| 12 | 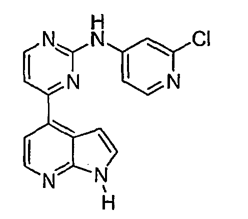 | MS *m*/*z* 323.07 (M + 1). |
| 13 | 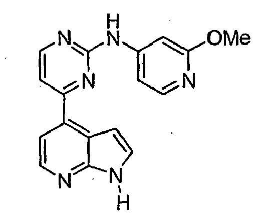 | MS *m*/*z* 319.13 (M + 1). |
| 14 | 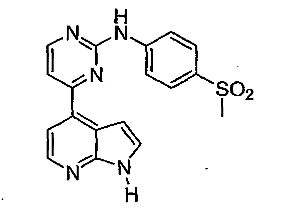 | MS *m*/*z* 366.21(M + 1). |
| 15 | 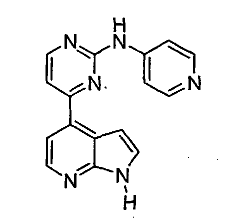 | MS *m*/*z* 289.11 (M + 1). |
| 16 | 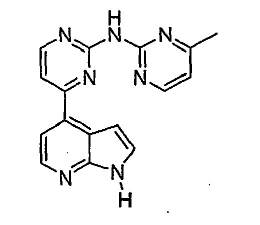 | MS *m*/*z* 304.3 (M + 1). |
| 17 | 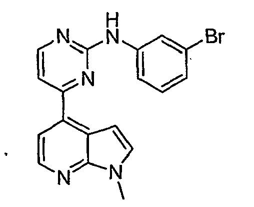 | MS *m*/*z* 380.04 (M + 1). |
| 18 | 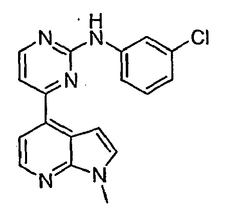 | MS *m*/*z* 336.21 (M + 1). |
| 19 | 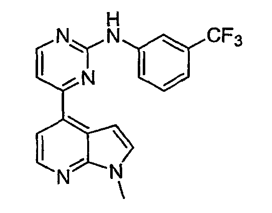 | MS *m*/*z* 370.15 (M + 1). |
| 20 | 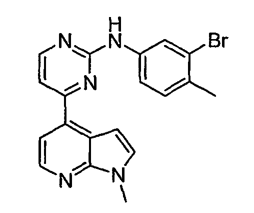 | MS *m*/*z* 394.04 (M + 1). |
| 21 | 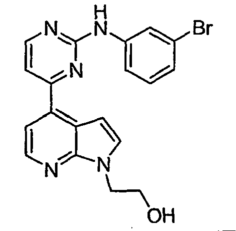 | MS *m*/*z* 410.15 (M + 1). |
| 22 | 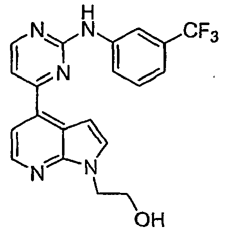 | MS *m*/*z* 400.13 (M + 1). |
| 23 | 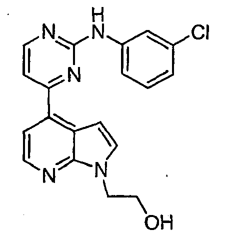 | MS *m*/*z* 366.20 (M + 1). |
| 24 | 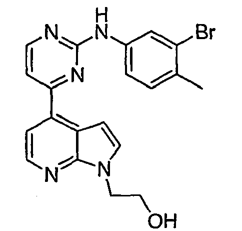 | MS *m*/*z* 424.10 (M + 1). |
| 25 | 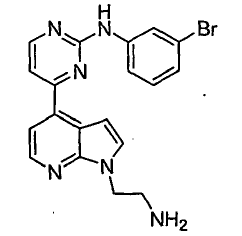 | MS *m*/*z* 409.18 (M + 1). |
| 26 | 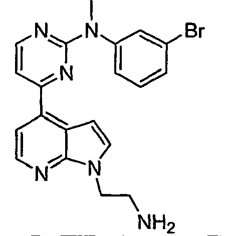 | MS *m*/*z* 423.21 (M + 1). |
| 27 | 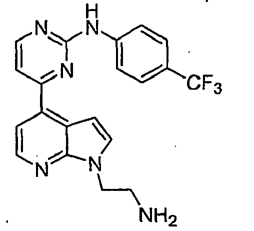 | MS *m*/*z* 399.25 (M + 1). |
| 28 | 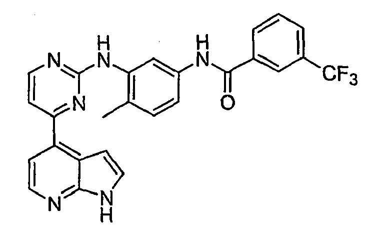 | MS *m*/*z* 489.14 (M + 1). |
| 29 | 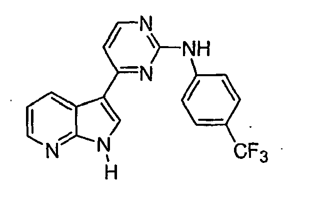 | δ 12.52 (s, 1H), 10.08 (s, 1H), 9.17 (*dd*, *J* = 4.8, 1.6 Hz, 1H), 8.72 (s, 1H), 8.65 (d, J = 5.2, 1H), 8.54 (*dd*, *J* = 4.8, 1.6 Hz, 1H), 8.26 (d, J = 8.4 Hz, 2H), 7.64 (*d*, *J* = 8.4 Hz, 2H), 7.63 (d, *J* = 5.6 Hz, 1H), 7.24 (dd, *J* = 7.6, 4.4 Hz, 1H); MS *m*/*z* 356.32 (M + 1). |
| 30 | 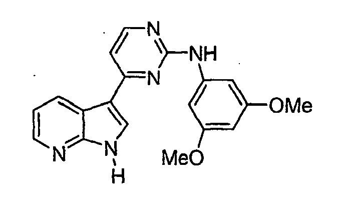 | δ 12.34 (s, 1H), 9.43 (s, 1H), 9.01 (*d*, *J* = 7.6 Hz, 1H), 8.50 (s, 1H), 8.42 (d, *J* = 5.2, 1H), 8.36 (*d*, *J* = 4.6 Hz, 1H), 7.37 (d, *J* = 5.6, 1H), 7.24 (*dd*, *J* = 8.0, 4.8 Hz, 1H), 7.16 (s, 2H), 6.19 (t, *J* = 2.0 Hz, 1H); MS *m*/*z* 348.37 (M + 1). |
| 31 | 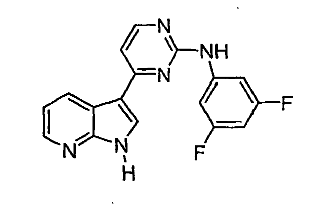 | MS *m*/*z* 324.09 (M + 1). |
| 32 | 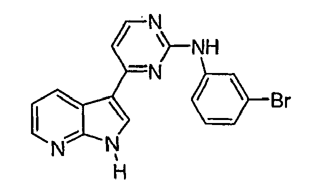 | MS *m*/*z* 366.02 (M + 1). |
| 33 | 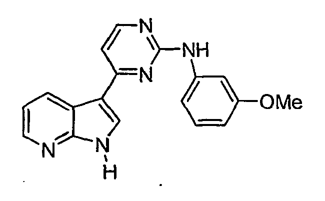 | MS *m*/*z* 318.14 (M + 1). |
| 34 | 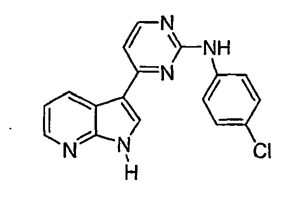 | MS *m*/*z* 322.01 (M + 1). |
| 35 | 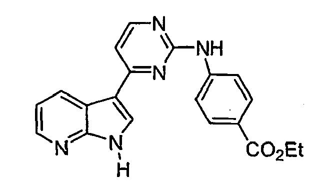 | MS *m*/*z* 360.14 (M+ 1). |
| 36 | 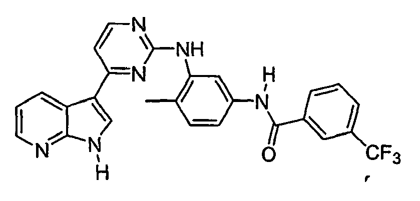 | MS *m*/*z* 489.33 (M + 1). |
| 37 | 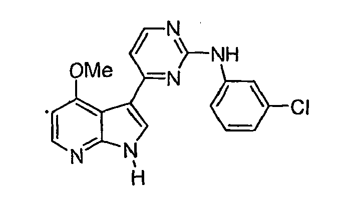 | MS *m*/*z* 352.21 (M + 1). |
| 38 | 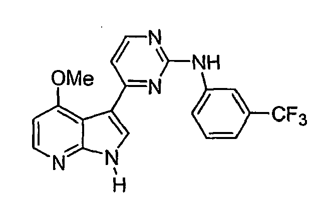 | MS *m*/*z* 386.32 (M + 1). |
| 39 | 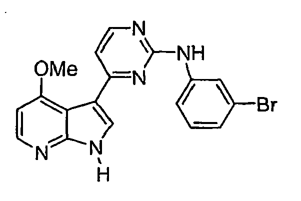 | MS *m*/*z* 396.09 (M + 1). |
| 40 | 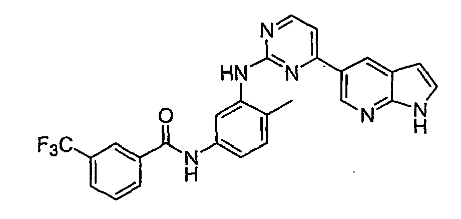 | MS *m*/*z* 489.21 (M + 1). |
| 41 | 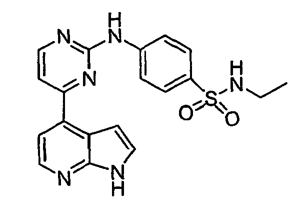 | MS *m*/*z* 395.01 (M + 1). |
| 42 | 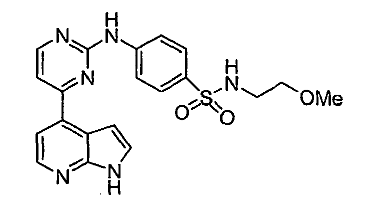 | MS *m*/*z* 425.12 (M + 1). |
| 43 | 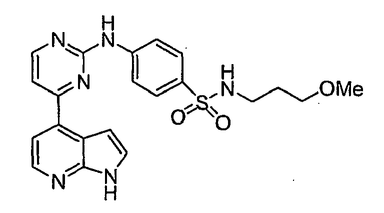 | MS *m*/*z* 439.13 (M + 1). |
| 44 | 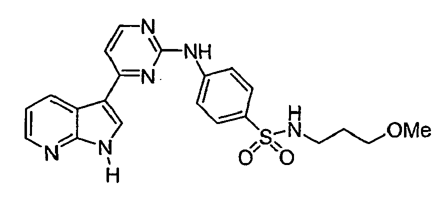 | MS *m*/*z* 439.15 (M + 1). |
| 45 | 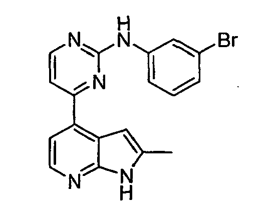 | MS *m*/*z* 439.15 (M + 1). |
| 46 | 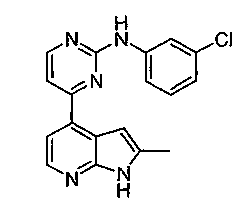 | MS *m*/*z* 336.21 (M + 1). |
| 47 | 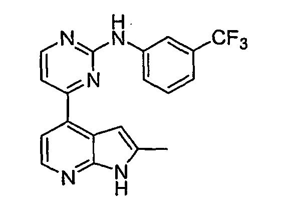 | MS *m*/*z* 370.12 (M + 1). |
| 48 | 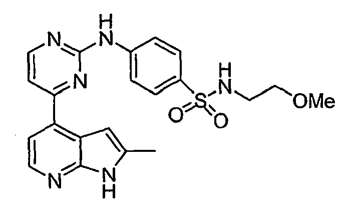 | MS *m*/*z* 439.14 (M + 1). |
| 49 | 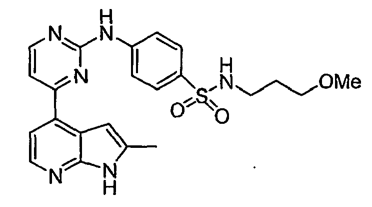 | MS *m*/*z* 453.16 (M + 1). |
| 50 | 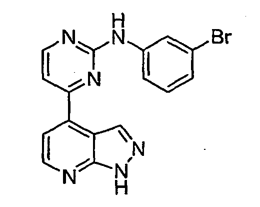 | MS *mlz* 367.01 (M + 1). |
| 51 | 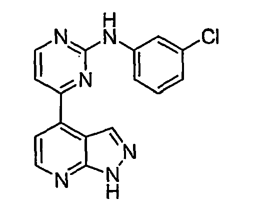 | MS *m*/*z* 323.05 (M + 1). |
| 52 | 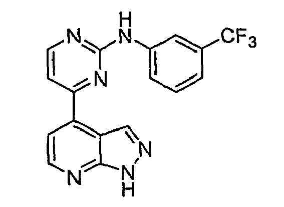 | MS *m*/*z* 357.12 (M + 1). |
| 53 | 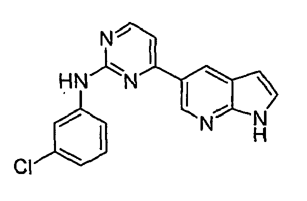 | MS *m*/*z* 322.14 (M + 1). |
| 54 | 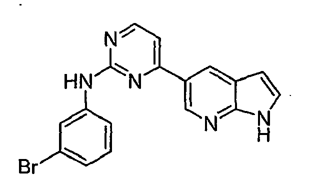 | MS *m*/*z* 366.21 (M + 1). |
| 55 | 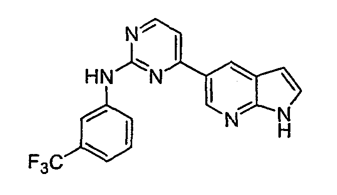 | MS *m*/*z* 356.12 (M + 1). |

### Assays

Compounds of the invention are assayed to measure their capacity to inhibit CDKs, Aurora, Jak2, Rock, CAMKII, FLT3, Tie2, TrkB, FGFR3 and KDR kinases.

### FGFR3 (Enzymatic Assay)

Kinase activity assay with purified FGFR3 (Upstate) is carried out in a final volume of 10 µL containing 0.25 µg/mL of enzyme in kinase buffer (30 mM Tris-HCl pH7.5, 15 mM MgCl₂, 4.5 mM MnCl₂, 15 µM Na₃VO₄ and 50 µg/mL BSA), and substrates (5 µg/mL biotin-poly-EY(Glu, Tyr) (CIS-US, Inc.) and 3µM ATP). Two solutions are made: the first solution of 5 µl contains the FGFR3 enzyme in kinase buffer was first dispensed into 384- format ProxiPlate® (Perkin-Elmer) followed by adding 50 nL of compounds dissolved in DMSO, then 5 µl of second solution contains the substrate (poly-EY) and ATP in kinase buffer was added to each wells. The reactions are incubated at room temperature for one hour, stopped by adding 10 µL of HTRF detection mixture, which contains 30 mM Tris-HCl pH7.5, 0.5 M KF, 50 mM ETDA, 0.2 mg/mL BSA, 15 µg/mL streptavidin-XL665 (CIS-US, Inc.) and 150 ng/mL cryptate conjugated anti-phosphotyrosine antibody (CIS-US, Inc.). After one hour of room temperature incubation to allow for streptavidin-biotin interaction, time resolved florescent signals are read on Analyst GT (Molecular Devices Corp.). IC₅₀ values are calculated by linear regression analysis of the percentage inhibition of each compound at 12 concentrations (1:3 dilution from 50 µM to 0.28 nM). In this assay, compounds of the invention have an IC₅₀ in the range of 10 nM to 2 µM.

### FGFR3 (Cellular Assay)

Compounds of the invention are tested for their ability to inhibit transformed Ba/F3-TEL-FGFR3 cells proliferation, which is depended on FGFR3 cellular kinase activity. Ba/F3-TEL-FGFR3 are cultured up to 800,000 cells/mL in suspension, with RPMI 1640 supplemented with 10% fetal bovine serum as the culture medium. Cells are dispensed into 384-well format plate at 5000 cell/well in 50 µL culture medium. Compounds of the invention are dissolved and diluted in dimethylsufoxide (DMSO). Twelve points 1:3 serial dilutions are made into DMSO to create concentrations gradient ranging typically from 10 mM to 0.05 µM. Cells are added with 50 nL of diluted compounds and incubated for 48 hours in cell culture incubator. AlamarBlue® (TREK Diagnostic Systems), which can be used to monitor the reducing environment created by proliferating cells, are added to cells at final concentration of 10%. After additional four hours of incubation in a 37 °C cell culture incubator, fluorescence signals from reduced AlamarBlue® (Excitation at 530 nm, Emission at 580 nm) are quantified on Analyst GT (Molecular Devices Corp.). IC₅₀ values are calculated by linear regression analysis of the percentage inhibition of each compound at 12 concentrations.

### FLT3 (Cellular Assay)

The effects of compounds of the invention on the cellular activity of FLT3 are conducted using identical methods as described above for FGFR3 cellular activity, except that Ba/F3-FLT3-ITD is used in stead of Ba/F3-TEL-FGFR3.

### Upstate KinaseProfiler^{™} - Radio-enzymatic filter binding assay

Compounds of the invention are assessed for their ability to inhibit individual members of the kinase panel. The compounds are tested in duplicates at a final concentration of 10 µM following this generic protocol. Note that the kinase buffer composition and the substrates vary for the different kinases included in the "Upstate KinaseProfiler^{™}" panel. Kinase buffer (2.5µL, 10x - containing MnCl₂ when required), active kinase (0.001-0.01 Units; 2.5µL), specific or Poly(Glu4-Tyr) peptide (5-500µM or .01mg/ml) in kinase buffer and kinase buffer (50µM; 5µL) are mixed in an eppendorf on ice. A Mg/ATP mix (10µL; 67:5 (or 33.75) mM MgCl₂, 450 (or 225) µM ATP and 1 µCi/µl [y-³²P]-ATP (3000Ci/mmol)) is added and the reaction is incubated at about 30°C for about 10 minutes. The reaction mixture is spotted (20µL) onto a 2cm x 2cm P81 (phosphocellulose, for positively charged peptide substrates) or Whatman No. 1 (for Poly (Glu4-Tyr) peptide substrate) paper square. The assay squares are washed 4 times, for 5 minutes each, with 0.75% phosphoric acid and washed once with acetone for 5 minutes. The assay squares are transferred to a scintillation vial, 5 ml scintillation cocktail are added and ³²P incorporation (cpm) to the peptide substrate is quantified with a Beckman scintillation counter. Percentage inhibition is calculated for each reaction.

Compounds of Formula I, in free form or in pharmaceutically acceptable salt form, exhibit valuable pharmacological properties, for example, as indicated by the *in vitro* tests described in this application. For example, compounds of Formula I preferably show an IC₅₀ in the range of 1 x 10⁻¹⁰ to 1 x 10⁻⁵ M, preferably less than 500nM, 400nM, 300nM and 200nM for at least one of the kinase listed in the kinase panel. Compounds of Formula I, at a concentration of 10µM, preferably show a percentage inhibition of greater than 50%, preferably greater than about 70%, against CDKs, Aurora, Jak2, Rock, CAMKII, FLT3, Tie2, TrkB, FGFR3 and/or KDR kinases.

## Claims

1. A compound selected from Formula Ia, Ib and Ic: 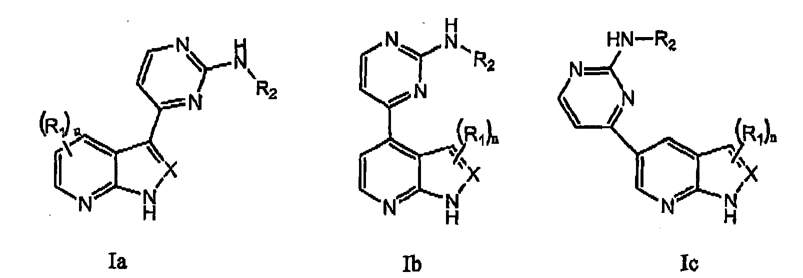 in which:
n is selected from 0, 1 and 2;
R₁ is selected from halo, C₁₋₆alkyl, C₁₋₆alkoxy, halo-substituted-C₁₋₆alkyl and halo-substituted-C₁₋₆alkoxy;
R₂ is selected from C₆₋₁₀aryl-C₀₋₄alkyl and C₅₋₁₀heteroaryl-C₀₋₄alkyl; wherein said aryl or heteroaryl of R₂ is optionally substituted by 1-3 radicals independently selected from halo, C₁₋₆alkyl, C₁₋₆alkoxy, halo-substituted-C₁₋₆alkyl, halo-substituted-C₁₋₆alkoxy, -S(O)₀₋₂R₅, -COOR₅, -C(O)NR₅R₆ and -NR₅C(O)R₆; wherein R₅ is selected from hydrogen and C₁₋₆alkyl; and R₆ is selected from C₆₋₁₀aryl and C₅₋₁₀heteroaryl; wherein said aryl or heteroaryl of R₆ is optionally substituted with 1 to 3 radicals independently selected from halo, C₁₋₆alkyl, C₁₋₆alkoxy, halo-substitated-C₁₋₆alkyl and halo-substituted-C₁₋₆alkoxy;
X is selected from CR₇ or N; wherein R₇ is selected from hydrogen and C₁₋₆alkyl; and the pharmaceutically acceptable salts, hydrates, and solvates thereof;
with the proviso that the compound is not

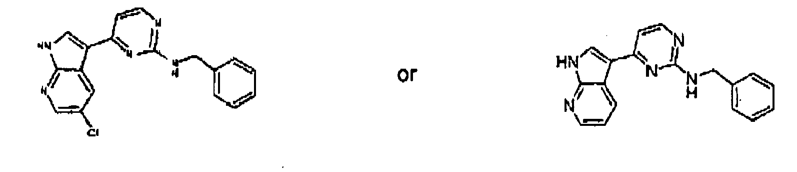

2. The compound of claim 1 in which;
n is selected from 0 and 1;
R₁ is C₁₋₆alkoxy;
R₂ is selected from C₆₋₁₀aryl-C₀₋₄alkyl and C₅₋₁₀heteroaryl-C₀₋₄alkyl;
wherein said aryl or heteroaryl of R₂ is optionally substituted by 1-3 radicals independently selected from halo, C₁₋₆alkyl, C₁₋₆alkoxy, halo-substituted-C₁₋₆alkyl, -S(O)₀₋₂R₅, -COOR_{5,} and -NR₅C(O)R₆; wherein R₅ is selected from hydrogen and C₁₋₆alkyl; and R₆ is C₆₋₁₀aryl optionally substituted with 1 to 3 radicals independently selected from halo-substituted-C₁₋₆alkyl.

3. The compound of claim 2 in which: R₁ is methoxy; R₂ is selected from phenyl, benzyl and pyridinyl; wherein said phenyl, benzyl or pyridinyl of R₂ is optionally substituted with 1 to 2 radicals independently selected from chloro, bromo, fluoro, methyl, trifluoromethoxy, trifluoromethyl, -COO₂R₅, -S(O)₂R₅ and -NHC(O)R₆; wherein R₅ is selected from methyl and ethyl; and R₆ is phenyl optionally substituted with trifluoromethyl.

4. The compound of claim 1 selected from (3-Chloro-phenyl)-[4-(1H-pyrrolo[2,3-b]pyridin-4-yl)-pyrimidin-2-yl]-amine; (4-Fluoro-phenyl)-[4-(1H-pyrrolo[2,3-b]pyridin-4-yl)-pyrimidin-2-yl]-amine; [4-(1H-Pyrrolo[2,3-b]pyridin-4-yl)-pyrimidin-2-yl]-(4-trifluoromethoxy-phenyl)-amine; [4-(1H-Pyrrolo[2,3-b]pyridin-4-yl)-pyrimidin-2-yl]-(3-trifluoromethyl-phenyl)-amine; (3,4-Difluoro-phenyl)-[4-(1H-pyrrolo[2,3-b]pyridin-4-yl)-pyrimidin-2-yl]-amine; [4-(1H-Pyrrolo[2,3-b]pyridin-4-yl)-pyrimidin-2-yl]-(4-trifluoxomethyl-phenyl)-amine; 4-[4-(1H-Pyrrolo[2,3-b]pyridin-4-yl)-pyrimidin-2-ylamino]-benzoic acid ethyl ester; (3-Methoxy-phenyl)-[4-(1H-pyrrolo[2,3-b]pyridin-4-yl)-pyrimidin-2-yl]-amine; (3-Fluoro-benzyl)-[4-(1H-pyrrolo[2,3-b]pyridin-4-yl)-pyrinfdin-2-yl]-amine; (3,5-Dimethoxy-phenyl)-(4-(1H-pyrrolo[2,3-b]pyridin-4-yl)pyrimidin-2-yl]-amine; (2-Methyl-pyridin-4-yl)-[4-(1H-pyrrolo[2,3-b]pyridin-4-yl)-pyrimidin-2-yl]-amine; (2-Chloro-pyridin-4-yl)-[4-(1H-pyrrolo[2,3-b]pyridin-4-yl)-pyrimidin-2-yl]-amine; (2-Methoxy-pyridin-4-yl)-[4-(1H-pyrrolo[2,3-b]pyridin-4-yl)-pyrimidin-2-yl]-amine; (4-Methanesulfonyl-phenyl)-[4-(1H-pyrrolo[2,3-b]pyridin-4-yl)-pyrimidin-2-yl]-amine; Pyridin-4-yl-[4-(1H-pyrrolo[2,3-b]pyridin-4-yl)-pyrimidin-2-yl]-amine; (4-Methyl-pyrimidin-2-yl)-[4-(1H-pyrrolo(2,3-b]pyridin-4-yl)-pyrimidin-2-yl]-amine; (3-Bromophenyl)-[4-(1-methyl-1H-pyrrolo[2,3-b]pyridin-4-yl)-pyrimidin-2-yl]-amine; (3-Chlorophenyl)-[4-(1-methyl-1H-pyrrolo[2,3-b]pyridin-4-yl)-pyrimidin-2-yl]-amine; [4-(1-Methyl-1H-pyrrolo[2,3-b]pyridin-4-yl)-pyrimidin-2-yl]-(3-trifluoromethyl-phenyl)-amine; (3-Bromo-4-methyl-phenyl)-[4-(1-methyl-1H-pyrrolo[2,3-b]pyridin-4-yl)-pyrimidin-2-yl]-amine; 2-{4-[2-(3-Bromo-phenylamino)-pyrimidin-4-yl]-pyrrolo[2,3-b]pyridin-1-yl}-ethanol; 2-{4-[2-(3-Trifluoromethyl-phenylamino)pyrimidin-4-yl]-pyrrolo[2,3-b]pyridin-1-yl}-ethanol; 2-{4-[2-(3-Chloro-phenylamino)-pyrimidin-4-yl]-pyrrolo[2,3-b]pyridin-1-yl}-ethanol; 2-{4-[2-(3-Bromo-4-methyl-phenylamino)-pyrimidin-4-yl]-pyrrolo[2,3-b]pyridin-1-yl}-ethanol; {4-[1-(2-Amino-ethyl)-1H-pyrrolo[2,3-b]pyridin-4-yl]-pyrimidin-2-yl}-(3-bromo-phenyl)-amine; {4-[1-(2-Amino-ethyl)-1H-pyrrolo[2,3-b]pyridin-4-yl]-pyrimidin-2-yl}-(3-bromo-phenyl)-methyl-amine; {4-[1-(2-Amino-ethyl)-1H-pyrrolo[2,3-b]pyridin-4-yl]-pyrimidin-2-yl}-(4-trifluoromethyl-phenyl)-amine; N-{4-Methyl-3-[4-(1H-pyrrolo[2,3-b]pyridin-4-yl)-pyrimidin-2-ylamino]-phenyl}-3-trifluoromethyl-benzamide; [4-(1H-Pyrrolo[2,3-b]pyridin-3-yl)-pyrimidin-2-yl]-(4-trifluoromethyl-phenyl)-amine; (3,5-Dimethoxy-phenyl)-[4-(1H-pyrrolo[2,3-b]pyridin-3-yl)-pyrimidin-2-yl]-amine; (3,5-Difluoro-phenyl)-[4-(1H-pyrrolo[2,3-b]pyridin-3-yl)-pyrimidin-2-yl]-amine; (3-Bromophenyl)-[4-(1H-pyrrolo[2,3-b]pyridin-3-yl)-pyrimidin-2-yl]-amine; (3-Methoxy-phenyl)-[4-(1H-pyrrolo[2,3-b]pyridin-3-yl)-pyrimidin-2-yl]-amine; (4-Chloro-phenyl)-[4-(1H-pyrrolo[2,3-b]pyridin-3-yl)-pyrimidin-2-yl]-amine; 4-[4-(1H-Pyrrolo[2,3-b]pyridin-3-yl)-pyrimidin-2-ylamino]-benzoic acid ethyl ester; N-{4-Methyl-3-[4-(1H-pyrrolo[2,3-b]pyridin-3-yl)-pyrimidin-2-ylamino]-phenyl}-3-trifluoromethyl-benzamide; (3-Chlorophenyl)-[4-(4-mothoxy-1H-pyrrolo[2,3-b]pyridin-3-yl)-pyrimidin-2-yl]-amine; [4-(4-Methoxy-1H-pyrrolo[2,3-b]pyridin-3-yl)-pyrimidin-2-yl]-(3-trifluoromethyl-phenyl)-amine; (3-Bromo-phenyl)-[4-(4-methoxy-1H-pyrrolo[2,3-b]pyridin-3-yl)-pyrimidin-2-yl]-amine; N-{4-Methyl-3-[4-(1H-pyrrolo[2,3-b]pyridin-5-yl)-pyrimidin-2-ylamino]-phenyl}-3-trifluoromethyl-benzamide; N-Ethyl-4-[4-(1H-pyrrolo[2,3-b]pyridin-4-yl)-pyrimidin-2-ylamino]-benzenesulfonamide; N-(2-Methoxy-ethyl)-4-[4-(1H-pyrrolo[2,3-b]pyridin-4-yl)-pyrimidin-2-ylamino]-benzenesulfonamide; N-(3-Methoxy-propyl)-4-[4-(1H-pyrrolo[2,3-b]pyridin-4-yl)-pyrimidin-2-ylamino]-benzenesulfonamide; N-(3-Methoxy-propyl)-4-[4-(1H-pyrrolo[2,3-b]pyridin-3-yl)-pyrimidin-2-ylamino]-benzenesulfonsmide; (3-Bromophenyl)-[4-(2-methyl-1H-pyrrolo[2,3-b]pyridin-4-yl)-pyrimidin-2-yl]-amine; (3-Chlorophenyl)-[4-(2-methyl-1H-pyrrolo[2,3-b]pyridin-4-yl)-pyrimedin-2-yl]-amine; [4-(2-Methyl-1H-pyrrolo[2,3-b]pyridin-4-yl)-pyrimidin-2-yl]-(3-trifluoromethyl-phenyl)-amine; N-(2-Methoxy-ethyl)-4-[4-(2-methyl-1H-pyrrolo[2,3-b]pyridin-4-yl)-pyrimidin-2-ylamino]-benzenesulfonamide; N-(3-Methoxy-propyl)-4-[4-(2-methyl-1H-pyrrolo[2,3-b]pyridin-4-yl)-pyrimidin-2-ylamino]-benzanesulfonamide; (3-Bromo-phenyl)-[4-(1H-pyrazolo[3,4-b)pyridin-4-yl)-pyrimidin-2-yl]-amine; (3-Chloro-phenyl)-[4-(1Hpyrazolo[3,4-b]pyridin-4-yl)-pyrimidin-2-yl]-amine; [4-(1H-Pyrazolo[3,4-b]pyridin-4-yl)-pyrimidin-2-yl]-(3-trifluoromethyl-phenyl)-amine; (3-Chloro-phenyl)-[4-(1H-pyrrolo[2,3-b]pyridin-5-yl)-pyrimidin-2-yl]-amine; (3-Bromo-phenyl)-[4-(1H-pyrrolo[2,3-b]pyridin-5-yl)-pyrimidin-2-y1]-amine; and [4-(1H-Pyrrolo[2,3-b]pyridin-5-yl)-pyrimidin-2-yl]-(3-trifluoromethylphenyl)-amine,

5. A pharmaceutical composition comprising a therapeutically effective amount of a compound of Claim 1 in combination with a pharmaceutically acceptable excipient.

6. A compound of claim 1 for use in treatment of a disease in which inhibition of kinase activity can prevent, inhibit or ameliorate the pathology and/or symptomology of the disease.

7. A compound according to claim 6, in which the kinase is selected from CDKs, Aurora, Jak 2, Rock, CAMKII, FLT3, Tie 2, TrkB, FGFR3 and KDR.

8. The use of a compound of claim 1 in the manufacture of a medicament for treating a disease in an animal in which the kinase activity of CDKs, Aurora, Jak2, Rock, CAMKII, FLT3, Tie2, TrkB, FGFR3 and KDR contributes to the pathology and/or symptomology of the disease.

## Patentansprüche

1. Verbindung, ausgewählt aus den Formel la, Ib und Ic: 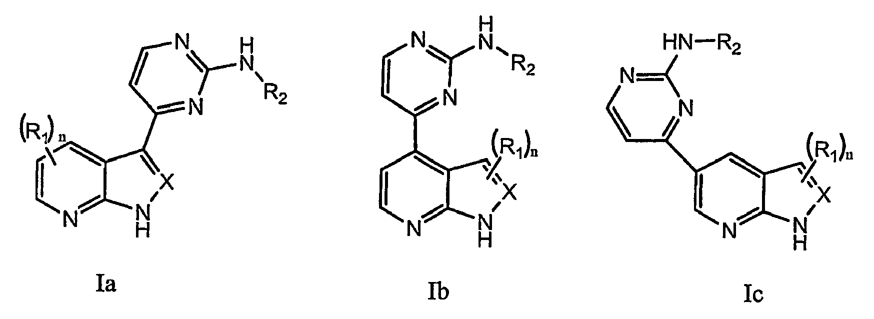 worin:
n aus 0, 1 und 2 ausgewählt ist;
R₁ aus Halogen, C₁₋₆-Alkyl, C₁₋₆-Alkoxy, halogensubstituiertem C₁₋₆-Alkyl und halogensubstituiertem C₁₋₆-Alkoxy ausgewählt ist;
R₂ aus C₆₋₁₀-Aryl-C₀₋₄-alkyl und C₅₋₁₀-Heteroaryl-C₀₋₄-alkyl ausgewählt ist; worin das Aryl oder Heteroaryl von R₂ gegebenenfalls mit 1-3 Resten substituiert ist, die unabhängig voneinander ausgewählt sind aus Halogen, C₁₋₆-Alkyl, C₁₋₆-Alkoxy, halogensubstituiertem C₁₋₆-Alkyl, halogensubstituiertem C₁₋₆-Alkoxy, -S(O)₀₋₂R₅, -COOR₅, -C(O)NR₅R₆ und -NR₅C(O)R₆; worin R₅ aus Wasserstoff und C₁₋₆-Alkyl ausgewählt ist; und R₆ aus C₆₋₁₀-Aryl und C₅₋₁₀-Heteroaryl ausgewählt ist; worin das Aryl oder Heteroaryl von R₆ gegebenenfalls mit 1 bis 3 Resten substituiert ist, die unabhängig voneinander aus Halogen, C₁₋₆-Alkyl, C₁₋₆-Alkoxy, halogensubstituiertem C₁₋₆-Alkyl und halogensubstituiertem C₁₋₆-Alkoxy ausgewählt sind;
X aus CR₇ und N ausgewählt ist; worin R₇ aus Wasserstoff und C₁₋₆-Alkyl ausgewählt ist; und pharmazeutisch annehmbare Salze, Hydrate und Solvate davon;
mit der Maßgabe, dass die Verbindung nicht 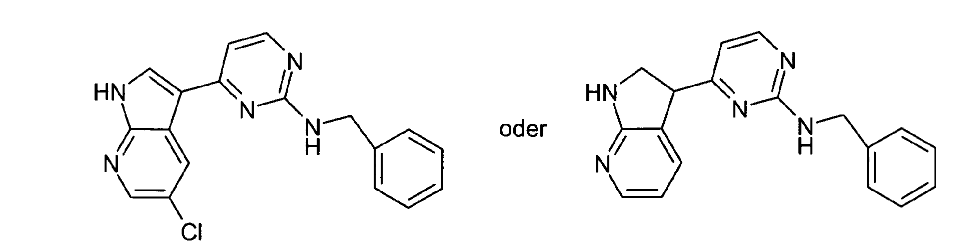
ist.

2. Verbindung nach Anspruch 1, worin
n aus 0 und 1 ausgewählt ist;
R₁ C₁₋₆-Alkoxy ist;
R₂ aus C₆₋₁₀-Aryl-C₀₋₄-alkyl und C₅₋₁₀-Heteroaryl-C₀₋₄-alkyl ausgewählt ist;
worin das Aryl oder Heteroaryl von R₂ gegebenenfalls mit 1-3 Resten substituiert ist, die unabhängig voneinander ausgewählt sind aus Halogen, C₁₋₆-Alkyl, C₁₋₆-Alkoxy, halogensubstituiertem C₁₋₆-Alkyl, -S(O)₀₋₂R₅, -COOR₅ und -NR₅C(O)R₆; worin R₅ aus Wasserstoff und C₁₋₆-Alkyl ausgewählt ist; und R₆ C₆₋₁₀-Aryl ist, das gegebenenfalls mit 1 bis 3 Resten substituiert ist, die unabhängig voneinander aus halogensubstituiertem C₁₋₆-Alkyl ausgewählt sind.

3. Verbindung nach Anspruch 2, worin R₁ Methoxy ist; R₂ aus Phenyl, Benzyl und Pyridinyl ausgewählt ist; worin das Phenyl, Benzyl oder Pyridinyl von R₂ gegebenenfalls mit 1 bis 2 Resten substituiert ist, die unabhängig voneinander aus Chlor, Brom, Fluor, Methyl, Trifluormethoxy, Trifluormethyl, -COO₂R₅, -S(O)₂R₅ und -NHC(O)R₆ ausgewählt ist; worin R₅ aus Methyl und Ethyl ausgewählt ist; und R₆ Phenyl ist, das gegebenenfalls mit Trifluormethyl substituiert ist.

4. Verbindung nach Anspruch 1, ausgewählt aus (3-Chlorphenyl)-[4-(1H-pyrrolo-[2,3-b]pyridin-4-yl)pyrimidin-2-yl]amin; (4-Fluorphenyl)-[4-(1H-pyrrolo[2,3-b]pyridin-4-yl)pyrimidin-2-yl]amin; [4-(1H-Pyrrolo[2,3-b]pyridin-4-yl)pyrimidin-2-yl]-(4-trifluormethoxyphenyl)amin; [4-(1H-Pyrrolo[2,3-b]pyridin-4-yl)pyrimidin-2-yl]-(3-trifluormethylphenyl)amin; (3,4-Difluorphenyl)-[4-(1H-pyrrolo[2,3-b]pyridin-4-yl)pyrimidin-2-yl]amin; [4-(1H-Pyrrolo[2,3-b]pyridin-4-yl)pyrimidin-2-yl]-(4-trifluormethyl-phenyl)amin; 4-[4-(1H-Pyrrolo[2,3-b]pyridin-4-yl)pyrimidin-2-ylamino]benzoesäureethylester; (3-Methoxyphenyl)-[4-(1H-pyrrolo[2,3-b]pyridin-4-yl)pyrimidin-2-yl]amin; (3-Fluorbenzyl)-[4-(1H-pyrrolo[2,3-b]pyridin-4-yl)pyrimidin-2-yl]amin; (3,5-Dimethoxyphenyl)-[4-(1H-pyrrolo[2,3-b]pyridin-4-yl)pyrimidin-2-yl]amin; (2-Methylpyridin-4-yl)-[4-(1H-pyrrolo-[2,3-b]pyridin-4-yl)pyrimidin-2-yl]amin; (2-Chlorpyridin-4-yl)-[4-(1H-pyrrolo[2,3-b]-pyridin-4-yl)pyrimidin-2-yl]amin; (2-Methoxypyridin-4-yl)-[4-(1H-pyrrolo[2,3-b]pyridin-4-yl)pyrimidin-2-yl]amin; (4-Methansulfonylphenyl)-[4-(1H-pyrrolo[2,3-b]pyridin-4-yl)-pyrimidin-2-yl]amin; Pyridin-4-yl-[4-(1H-pyrrolo[2,3-b]pyridin-4-yl)pyrimidin-2-yl]amin; (4-Methylpyrimidin-2-yl)-[4-(1H-pyrrolo[2,3-b]pyridin-4-yl)pyrimidin-2-yl]amin; (3-Bromphenyl)-[4-(1-methyl-1H-pyrrolo[2,3-b]pyridin-4-yl)pyrimidin-2-yl]amin; (3-Chlorphenyl)-[4-(1-methyl-1H-pyrrolo[2,3-b]pyridin-4-yl)pyrimidin-2-yl]amin; [4-(1-Methyl-1H-pyrrolo[2,3-b]pyridin-4-yl)pyrimidin-2-yl]-(3-trifluormethylphenyl)amin; (3-Brom-4-methylphenyl)-[4-(1-methyl-1H-pyrrolo[2,3-b]pyridin-4-yl)pyrimidin-2-yl]amin; 2-{4-[2-(3-Bromphenylamino)pyrimidin-4-yl]pyrrolo[2,3-b]pyridin-1-yl}ethanol; 2-{4-[2-(3-Tri-fluormethylphenylamino)pyrimidin-4-yl]pyrrolo[2,3-b]pyridin-1-yl}ethanol; 2-{4-[2-(3-Chlorphenylamino)pyrimidin-4-yl]pyrrolo[2,3-b]pyridin-1-yl}ethanol; 2-{4-[2-(3-Brom-4-methylphenylamino)pyrimidin-4-yl]pyrrolo[2,3-b]pyridin-1-yl}ethanol; {4-[1-(2-Aminoethyl)-1H-pyrrolo[2,3-b]pyridin-4-yl]pyrimidin-2-yl}-(3-bromphenyl)amin; {4-[1-(2-Aminoethyl)-1H-pyrrolo[2,3-b]pyridin-4-yl]pyrimidin-2-yl}-(3-bromphenyl)methylamin; {4-[1-(2-Aminoethyl)-1H-pyrrolo[2,3-b]pyridin-4-yl]pyrimidin-2-yl}-(4-trifluormethylphenyl)amin; N-{4-Methyl-3-[4-(1H-pyrrolo[2,3-b]pyridin-4-yl)pyrimidin-2-ylamino]-phenyl}-3-trifluormethylbenzamid; [4-(1H-Pyrrolo[2,3-b]pyridin-3-yl)pyrimidin-2-yl]-(4-trifluormethylphenyl)amin; (3,5-Dimethoxyphenyl)-[4-(1H-pyrrolo[2,3-b]pyridin-3-yl)-pyrimidin-2-yl]amin; (3,5-Difluorphenyl)-[4-(1H-pyrrolo[2,3-b]pyridin-3-yl)pyrimidin-2-yl]amin; (3-Bromphenyl)-[4-(1H-pyrrolo[2,3-b]pyridin-3-yl)pyrimidin-2-yl]amin; (3-Methoxyphenyl)-[4-(1H-pyrrolo[2,3-b]pyridin-3-yl)pyrimidin-2-yl]amin; (4-Chlorphenyl)-[4-(1H-pyrrolo[2,3-b]pyridin-3-yl)pyrimidin-2-yl]amin; 4-[4-(1H-Pyrrolo[2,3-b]pyridin-3-yl)pyrimidin-2-ylamino]benzoesäureethylester; N-{4-Methyl-3-[4-(1H-pyrrolo[2,3-b]-pyridin-3-yl)pyrimidin-2-ylamino]phenyl}-3-trifluormethylbenzamid; (3-Chlorphenyl)-[4-(4-methoxy-1H-pyrrolo[2,3-b]pyridin-3-yl)pyrimidin-2-yl]amin; [4-(4-Methoxy-1H-pyrrolo[2,3-b]pyridin-3-yl)pyrimidin-2-yl]-(3-trifluormethylphenyl)amin; (3-Bromphenyl)-[4-(4-methoxy-1H-pyrrolo[2,3-b]pyridin-3-yl)pyrimidin-2-yl]amin; N-{4-Methyl-3-[4-(1H-pyrrolo[2,3-b]pyridin-5-yl)pyrimidin-2-ylamino]phenyl}-3-trifluormethylbenzamid; N-Ethyl-4-[4-(1H-pyrrolo[2,3-b]pyridin-4-yl)pyrimidin-2-ylamino]benzolsulfonamid; N-(2-Methoxyethyl)-4-[4-(1H-pyrrolo[2,3-b]pyridin-4-yl)pyrimidin-2-ylamino]-benzolsulfonamid; N-(3-Methoxypropyl)-4-[4-(1H-pyrrolo[2,3-b]pyridin-4-yl)pyrimidin-2-ylamino]benzolsulfonamid; N-(3-Methoxypropyl)-4-[4-(1H-pyrrolo[2,3-b]pyridin-3-yl)pyrimidin-2-ylamino]benzolsulfonamid; (3-Bromphenyl)-[4-(2-methyl-1H-pyrrolo-[2,3-b]pyridin-4-yl)pyrimidin-2-yl]amin; (3-Chlorphenyl)-[4-(2-methyl-1H-pyrrolo[2,3-b]-pyridin-4-yl)pyrimidin-2-yl]amin; [4-(2-Methyl-1H-pyrrolo[2,3-b]pyridin-4-yl)pyrimidin-2-yl]-(3-trifluormethylphenyl)amin; N-(2-Methoxyethyl)-4-[4-(2-methyl-1H-pyrrolo[2,3-b]-pyridin-4-yl)pyrimidin-2-ylamino]benzolsulfonamid; N-(3-Methoxypropyl)-4-[4-(2-methyl-1H-pyrrolo[2,3-b]pyridin-4-yl)pyrimidin-2-ylamino]-benzolsulfonamid; (3-Bromphenyl)-[4-(1H-pyrazolo[3,4-b]pyridin-4-yl)pyrimidin-2-yl]amin; (3-Chlorphenyl)-[4-(1H-pyrazolo[3,4-b]pyridin-4-yl)pyrimidin-2-yl]amin; [4-(1H-Pyrazolo[3,4-b]pyridin-4-yl)-pyrimidin-2-yl]-(3-trifluormethylphenyl)amin; (3-Chlorphenyl)-[4-(1H-pyrrolo[2,3-b]-pyridin-5-yl)pyrimidin-2-yl]amin; (3-Bromphenyl)-[4-(1H-pyrrolo[2,3-b]pyridin-5-yl)-pyrimidin-2-yl]amin; und [4-(1H-Pyrrolo[2,3-b]pyridin-5-yl)pyrimidin-2-yl]-(3-trifluormethylphenyl)amin.

5. Pharmazeutische Zusammensetzung, die eine therapeutisch wirksame Menge einer Verbindung nach Anspruch 1 in Kombination mit einem pharmazeutisch annehmbaren Exzipienten umfasst.

6. Verbindung nach Anspruch 1 zur Verwendung bei der Behandlung einer Erkrankung, bei der die Hemmung eines Kinaseantikörpers die Manifestation und/oder Symptomatik der Erkrankung verhindern, hemmen oder lindern kann.

7. Verbindung nach Anspruch 6, worin die Kinase aus CDKs, Aurora, Jak2, Rock, CAMKII, FLT3, Tie2, TrkB, FGFR3 und KDR ausgewählt ist.

8. Verwendung einer Verbindung nach Anspruch 1 bei der Herstellung eines Medikaments zur Behandlung einer Erkrankung bei einem Tier, bei dem die Kinaseaktivität von CDKs, Aurora, Jak2, Rock, CAMKII, FLT3, Tie2, TrkB, FGFR3 und KDR zur Manifestation und/oder Symptomatik der Erkrankung beiträgt.

## Revendications

1. Composé choisi parmi les formules Ia, Ib et Ic : 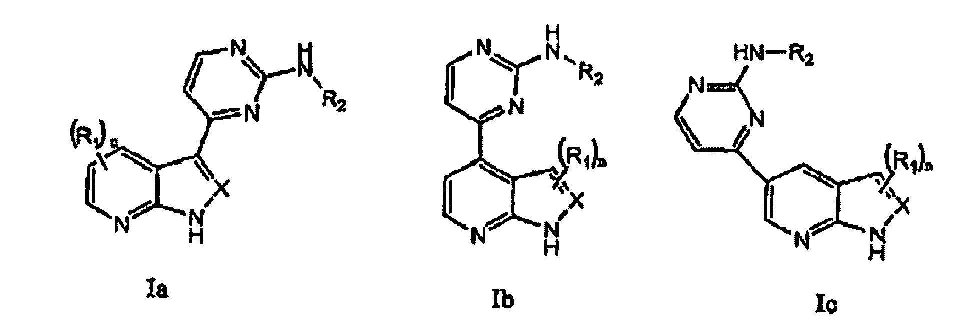 dans lesquelles :
n est choisi parmi 0, 1 et 2 ;
R₁ est choisi parmi les groupes halogéno, alkyle en C₁ à C₆, alcoxy en C₁ à C₆, alkyle en C₁ à C₆ substitué par halogéno et alcoxy en C₁ à C₆ substitué par halogéno ;
R₂ est choisi parmi les groupes aryle en C₆ à C₁₀-alkyl en C₀ à C₄ et hétéroaryl en C₅ à C₁₀-alkyle en C₀ à C₄ ; où ledit groupe aryle ou hétéroaryle de R₂ est facultativement substitué par 1 à 3 radicaux indépendamment choisis parmi les groupes halogéno, alkyle en C₁ à C₆, alcoxy en C₁ à C₆, alkyle en C₁ à C₆ substitué par halogéno, alcoxy en C₁ à C₆ substitué par halogéno, -S(O)₀₋₂R₅, -COOR₅, -C(O)NR₅R₆ et -NR₅C(O)R₆ ; où R₅ est choisi parmi un atome d'hydrogène et un groupe alkyle en C₁ à C₆ ; et R₆ est choisi parmi les groupes aryle en C₆ à C₁₀ et hétéroaryle en C₅ à C₁₀ ; où ledit groupe aryle ou hétéroaryle de R₆ est facultativement substitué par 1 à 3 radicaux indépendamment choisis parmi les groupes halogéno, alkyle en C₁ à C₆, alcoxy en C₁ à C₆, alkyle en C₁ à C₆ substitué par halogéno et alcoxy en C₁ à C₆ substitué par halogéno ;
X est choisi parmi CR₇ ou N ; où R₇ est choisi parmi un atome d'hydrogène et un groupe alkyle en C₁ à C₆ ; et leurs sels, hydrates et solvates pharmaceutiquement acceptables ;
à condition que le composé ne soit pas

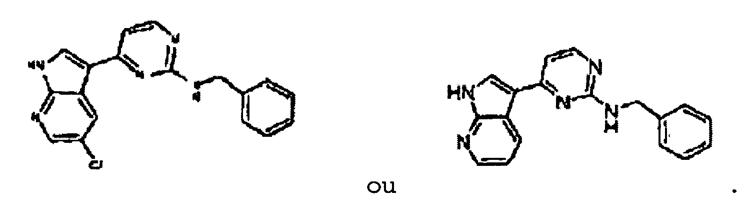

2. Composé selon la revendication 1, dans lequel :
n est choisi parmi 0 et 1 ;
R₁ est un groupe alcoxy en C₁ à C₆ ;
R₂ est choisi parmi les groupes aryl en C₅ à C₁₀-alkyle en C₀ à C₄ et hétéroaryl en C₅ à C₁₀-alkyle en C₀ à C₄ ; où ledit groupe aryle ou hétéroaryle de R₂ est facultativement substitué par 1 à 3 radicaux indépendamment choisis parmi les groupes halogéno, alkyle en C₁ à C₆, alcoxy en C₁ à C₆, alkyle en C₁ à C₆ substitué par halogéno, -S(O)₀₋₂R₅, -COOR₅, et - NR₅C(O)R₆ ; où R₅ est choisi parmi un atome d'hydrogène et un groupe alkyle en C₁ à C₆ ; et R₆ est choisi un groupe aryle en C₆ à C₁₀ facultativement substitué par 1 à 3 radicaux indépendamment choisis parmi un groupe alkyle en C₁ à C₆ substitué par halogéno.

3. Composé selon la revendication 2, dans lequel :
R₁ est un groupe méthoxy ; R₂ est choisi parmi les groupes phényle, benzyle et pyridinyle ; où ledit groupe phényle, benzyle ou pyridinyle de R₂ est facultativement substitué par 1 à 2 radicaux indépendamment choisis parmi les groupes chloro, bromo, fluoro, méthyle, trifluorométhoxy, trifluorométhyle, -COO₂R₅, -S(O)₂R₅ et -NHC(O)R₆ ; où R₅ est choisi parmi les groupes méthyle et éthyle ; et R₆ est un groupe phényle facultativement substitué par un groupe trifluorométhyle.

4. Composé selon la revendication 1, choisi parmi la (3-chloro-phényl)-[4-(1H-pyrrolo[2,3-b]pyridin-4-yl)-pyrimidin-2-yl]-amine ; la (4-fluoro-phényl)-[4-(1H pyrrolo[2,3-b]pyridin-4-yl)-pyrimidin-2-yl]-amine ; la [4-{1H-pyrrolo[2,3-5]pyridin-4-yl)-pyrimidin-2-yl]-{4-trifluorométhoxy-phényl)-amine ; la [4-(1H-pyrrolo[2,3-b]pyridin-4-yl)-pyrimidin-2-yl]-(3-trifluorométhyl-phényl)-amine ; la (3,4-difluoro-phényl)-[4-(1H-pyrrolo[2,3-b]pyridin-4-yl)-pyrimidin-2-yl]-amine ; la [4-(1H-pyrrolo[2,3-b]pyridin-4-yl)-pyrimidin-2-yl]-{4-trifluorométhyl-phényl)-amine ; l'ester d'éthyle d'acide 4[4-(1H-pyrrolo[2,3-b]pyridin-4-yl)-pyrimidin-2-ylamino)-benzoïque, la (3-méthoxy-phényl)-[4-(1H-pyrrolo[2,3-b]pyridin-4-yl)-pyrimidin-2-yl]-amine ; la (3-fluoro-benzyl)-[4-(1H-pyrrolo[2,3-b]pyridin-4-yl)-pyrimidin-2-yl]-amine ; la (3,5-diméthoxy-phényl)-[4-(1H-pyrrolo[2,3-b]pyridin-4-yl)-pyrimidin-2-yl]-amine ; la (2-méthyl-pyridin-4-y])-[4-(1H-pyrrolo[2,3-b]pyridin-4-yl)-pyrimidin-2-yl]-amine ; la (2-chloro-pyridin-4-yl)-[4-(1H-pyrrolo[2,3-b]pyridin-4-yl)-pyrimidin-2-yl]-amine ; la (2-méthoxy-pyridin-4-yl)-[4-(1H-pyrrolo[2,3-b]pyridin-4-yl)-pyrimidin-2-yl]-amine ; la (4-méthanesulfonyl-phényl)-[4-(1H-pyrrolo[2,3-b]pyridin-4yl)-pyrimidin-2-yl]-amine ; la pyridin-4-yl-[4-(1H-pyrrolo[2,3-b]pyridin-4-yl)pyrimidin-2-yl]-amine ; la (4-méthyl-pyrimidin-2-yl)-[4-(1H-pyrrolo[2,3-b]pyridin-4-yl)-pyrimidin-2-yl]-amine ; la (3-bromophényl)-[4-(1-méthyl-1H-pyrrolo[2,3-b]pyridin-4yl)-pyrimidin-2-yl]-amine ; la (3-chloro-phényl)-[4-(1-méthyl-1H-pyrrolo[2,3-b]pyridin-4-yl)-pyrimidin-2-yl]-amine ; la [4-(1-méthyl-1H-pyrrolo[2,3-b]pyridin-4-yl)-pyrimidin-2-yl]-(3-trifluorométhyl-phényl)-amine ; la (3-bromo-4-méthyl-phényl)-[4-(1-méthyl-1H-pyrrolo[2,3-b]pyridin-4-yl)-pyrimidin-2-yl]-amine ; le 2-{4-[2{3-bromo-phénylamino)pyrimidin-4-yl]-pyrrolo[2,3-b]pyridin-1-yl}-éthanol ; le 2-{4-[2-(3-trifluorométhyl-phénylamino)-pyrimidin-4-yl-pyrrolo[2,3-b]pyridin-1-yl}-éthanol ; le 2-{4-[2-(3-chloro-phénylamino)-pyrimidin-4-yl)-pyrrolo[2,3-b]pyridin-1-yl}-éthanol ; le 2-{4-[2-(3-bromo-4-méthyl-phénylamino)-pyrimidin-4-yl]-pyrrolo[2,3-b)pyridin-1-yl}-éthanol ; la (4-[1-(2-amino-éthyl)-1H-pyrrolo[2,3-b]pyridin-4-yl]-pyridin-2-yl}-(3-bromo-phényl)-amine ; la (4-[1-(2-amino-éthyl)-1H-pyrrolo[2,3-b]pyridin-4-yl)-pyrimidin-2-yl)-(3-bromo-phényl)-méthyl-amine ; la (4-[1-(2-amino-éthyl)-1H-pyrrolo[2,3-b]pyridin-4-yl]-pyrimidin-2-yl}-(4-trifluorométhyl-phényl)-amine ; le N-(4-méthyl-3-[4-(1H-pyrrolo[2,3-b]pyridin-pyrimidin-2-ylamino]-phényl}-3-trifluorométhyl-benzamide ; la [4(1H-pyrrolo[2,3-b]pyridin-3-yl)-pyrimidin-2-yl]-(4-trifluorométhyl-phényl)-amine ; la (3,5-diméthoxy-phényl)-(4-(1H-pyrrolo[2,3-b]pyridin-3-yl)-pyrimidin-2-yl]-amine ; la (3,5-difluoro-phényl)-[4-(1H-pyrrolo[2,3-b]pyridin-3-yl}pyrimidin-2-yl]-amine ; la (3-bromo-phényl)-[4-(1H-pyrrolo[2,3-b]pyridin-3-yl)-pyrimidin-2-yl]-amine ; la (3-méthoxy-phényl)-[4-(1H-pyrrolo(2,3-b]pyridin-3-yl)-pyrimidin-2-yl]-amine ; la (4-chlorophényl)-[4-(1H-pyrrolo[2,3-b]pyridin-3-yl)-pyrimidin-2-yl]-amine ; l'ester d'éthyle d'acide 4-[4-(1H-pyrrolo[2,3-b]pyridin-3-yl)-pyrimidin-2-ylaminol-benzoïque ; le N-(4-méthyl-3-[4-(1H-pyrrolo[2,3-b]pyridin-3-yl)-pyrimidin-2-ylamino]-phényl}-3-trifluorométhyl-benzamide ; la (3-chloro-phényl)-[4(4-méthoxy-1H-pyrrolo[2,3-b]pyridin-3-yl-pyrimidin-2-yl]-amine ; la [4-(4-méthoxy-1H-pyrrolo[2,3-b]pyridin-3-yl}-pyrimidin-2-yl]-(3-trifluorométhyl-phényl)-amine ; la (3-bromo-phényl)-[4-(4-méthoxy-1H-pyrrolo[2,3-b]pyridin-3-yl)-pyrimidin-2-yl]-amine ; le N-{4-méthyl-3-[4-(1H-pyrrolo[2,3-b]pyridin-5-yl)-pyrimidin-2-ylamino)phényl}-3-trifluorométhyl-benzamide ; le N-éthyl-4-[4-(1H-pyrrolo[2,3-b}pyridin-4-yl)-pyrimidin-2-ylamino]-benzènesulfonamide ; le N-(2-méthoxy-éthyl)-4-[4-(1H-pyrrolo[2,3-b]pyridin-4-yl)-pyrimidin-2-ylamino]-benzènesulfonamide ; le N-(3-méthoxy-propyl)-[4-(1H-pyrrolo[2,3-b]pyridin-4-yl)-pyrimidin-2-ylamino]-benzènesulfonamide ; le N-(3-méthoxy-propyl)-4-[4-(1H-pyrrolo[2,3-b]pyridin-3-yl)-pyrimidin-2-ylamino]-benzènesulfonamide ; la (3-bromo-phényl)-[4-(2-méthyl-1H-pyrrolo[2,3-b]pyridin-4-yl)-pyrimidin-2-yl]-amine ; la (3-chloro-phényl)-[4-(2-méthyl-1H-pyrrolo[2,3-b]pyridin-4-yl)-pyrimidin-2-yl]-amine ; la [4-(2-méthyl-1H-pyrrolo[2,3-b]pyridin-4-yl)-pyrimidin-2-yl]-3-trifluorométhyl-phényl)-amine ; le N-(2-méthoxy-éthyl)-4-[4-(2-méthyl-1H-pyrrolo[2,3-b]pyridin-4-yl)pyrimidin-2-ylamino]-benzènesulfonamide ; le N-(3-méthoxy-propyl)-4-[4-(2-méthyl-1H-pyrrolo[2,3-b]pyridin-4-yl)-pyrimidin-2-ylamino]benzènesulfonamide ; la (3-bromo-phényl)-[4-(1H-pyrazolo[3,4-pyridin-4-yl)-pyrimidin-2-yl]-amine ; la (3-chloro-phényl)-[4-(1H-pyrimidin-2-yl]-amine ; la [4(1H-pyrazolo[3,4-b]pyridin-4-yl)-pyrimidin-2-yl]-3-trifluorométhyl-phényl)-amine ; la (3-chloro-phényl)-[4-(1H-pyrrolo[2,3-b]pyridin-5-yl)-pyrimidin-2-yl]-amine ; la (3-bromo-phényl)-[4-(1H-pyrrolo[2,3-b]pyridin-5-yl)-pyrimidin-2-yl]-amine ; et la [4-(1H-pyrrolo[2,3-b]pyridin-5-yl)-pyrimidin-2-yl]-(3-trifluorométhyl-phényl)-amine.

5. Composition pharmaceutique comprenant une quantité thérapeutiquement efficace d'un composé selon la revendication 1 en combinaison avec un excipient pharmaceutiquement acceptable.

6. Composé selon la revendication 1 à utiliser dans le traitement d'une maladie où l'inhibition de l'activité de kinase peut prévenir, inhiber ou améliorer la pathologie et/ou symptomatologie de la maladie.

7. Composé selon la revendication 6, dans lequel la kinase est choisie parmi CDKs, Aurora, Jak2, Rock, CAMKII, FLT3, Tie2, TrkB, FGFR3 et KDR.

8. Utilisation d'un composé selon la revendication 1 dans la fabrication d'un médicament destiné au traitement d'une maladie chez un animal dans lequel l'activité de kinase de CDKs, Aurora, Jak2, Rock, CAMKII, FLT3, Tie2, TrkB, FGFR3 et KDR contribue à la pathologie et/ou symptomatologie de la maladie.
